# EUROPEAN PATENT SPECIFICATION

(11) **EP 1 931 771 B1**
(45) Date of publication and mention of the grant of the patent: **07.10.2015**
(21) Application number: 06820735.6
(22) Date of filing: 28.08.2006
(51) Int. Cl.: C12N 9/12

(54) **PRODUCTION OF GLOBOSIDES OLIGOSACCHARIDES USING METABOLICALLY ENGINEERED MICROORGANISMS**
HERSTELLUNG VON GLOBOSID-OLIGOSACCHARIDEN UNTER VERWENDUNG METABOLISCH MANIPULIERTER MIKROORGANISMEN
PRODUCTION D'OLIGOSACCHARIDES GLOBOSIDES A L'AIDE DE MICRO-ORGANISMES METABOLIQUEMENT MODIFIES

(30) Priority: 26.08.2005 US 711406 P
(43) Date of publication of application: 18.06.2008
(73) Proprietor: CENTRE NATIONAL DE LA RECHERCHE SCIENTIFIQUE -CNRS-, 75016 Paris (FR)
(72) Inventor: RANDRIANTSOA, Mialy, F-38400 Saint Martin d'Heres (FR); DROUILLARD, Sophie, F-38640 Claix (FR); SAMAIN, Eric, 38160 Gières (FR)
(74) Representative: Regimbeau
(86) International application number: PCT/IB2006/002248
(87) International publication number: WO 2007/023348

(56) References cited:
- SHAO JUN ET AL: "Overexpression and biochemical characterization of beta-1,3-N-acetylgalactosaminyltransferase LgtD from Haemophilus influenzae strain Rd" BIOCHEMICAL AND BIOPHYSICAL RESEARCH COMMUNICATIONS, vol. 295, no. 1, July 2002 (2002-07), pages 1-8, XP002424205 ISSN: 0006-291X
- SHAO JUN ET AL: "Donor substrate regeneration for efficient synthesis of globotetraose and isoglobotetraose." APPLIED AND ENVIRONMENTAL MICROBIOLOGY, vol. 68, no. 11, November 2002 (2002-11), pages 5634-5640, XP002424206 ISSN: 0099-2240
- HOOD DEREK W ET AL: "Genetic basis for expression of the major globotetraose-containing lipopolysaccharide from H. influenzae strain Rd (RM118)" GLYCOBIOLOGY, vol. 11, no. 11, November 2001 (2001-11), pages 957-967, XP002424207 ISSN: 0959-6658
- RYU KANG ET AL: "Synthesis of complex carbohydrates and glyconjugates - Enzymatic synthesis of globotetraose using beta-1,3-N-acetylgalactosaminyltrans ferase LgtD from Haemophilus infuenzae Strain Rd" METHODS IN MOLECULAR BIOLOGY HUMANA PRESS INC, 999 RIVERVIEW DR, STE 208, TOTOWA, NJ 07512-1165 USA SERIES : METHODS IN MOLECULAR BIOLOGY (ISSN 1064-3745(PRINT)), 2005, pages 93-105, XP009080452 ISSN: 1-58829-399-8(H)

### SEQUENCE LISTING

<110> Centre National de la Recherche Scientifique (CNRS) SAMAIN, Eric RANDRIANTSOA, Mialy DROUILLARD, Sophie
<120> Production of globosides oligosaccarides using metabolically engineered microorganisms
<130> D23745
<150> US 60/711,406
   <151> 2005-08-26
<160> 9
<170> PatentIn version 3.3
<210> 1
   <211> 933
   <212> DNA
   <213> Neisseria meningitidis
<220>
<230> LgtC gene of Neisseria meningitidis (GenBank U65788)
<400> 1
   atggacatcg tatttgcggc agacgacaac tatgccgcct atctttgcgt tgcggcaaaa      60
   agcgtggaag cggcccatcc cgatacggaa atcaggttcc acgtcctcga tgccggcatc     120
   agtgaggcaa accgggcggc ggttgctgcc aatttgcggg gggggggtaa tatccgcttt     180
   atagacgtaa accccgaaga tttcgccggc ttccccttaa acatcaggca catttccatc     240
   acgacttatg cccgcttgaa attgggcgaa tacattgccg attgcgataa agtcctgtat     300
   ctggatatag acgtattggt cagggacagc ctgaagccct tatgggatac cgatttgggc     360
   gataactggc ttggcgcgtg catcgatttg tttgtcgaaa ggcagaatgc ttacaaacaa     420
   aaaatcggta tggcggacgg agaatattat ttcaatgccg gcgtattgct gatcaacctg     480
   aaaaagtggc ggcagcacga tattttcaaa atggcctgcg aatgggtgga acaatacaag     540
   gacgtgatgc aatatcagga tcaggatatt ttgaacgggc tgtttaaagg cggggtgtgt     600
   tatgcgaaca gccgtttcaa ctttatgccg accaatgatg cctttatggc gaacaggttt     660
   gcgtcccgcc ataccgaccc gctttaccgc gaccggactt atacggcgat gcctgtcgcc     720
   gtcagccatt attgcggccc ggcaaagccg tggcacaggg actgcaccgc gtggggtgcg     780
   gaacgtttca cagaattggc gggcagcctg acgagcgttc ccgaagaatg gcgcggcaaa     840
   cttgccgtcc cgcaccgtgt gtttccgaca aagcgtatgc ttcaaagatg gcgcagaaag     900
   ctgtctgcca gattcttacg caagatttat tga                                  933
<210> 2
   <211> 323
   <212> PRT
   <213> Neisseria meningitidis
<220>
<230> LgtC protein of Neisseria meningitidis (id AAB48385)
<400> 2
   Met Glu Asn Cys Pro Leu Val Ser Val Ile Val Cys Ala Tyr Asn Ala
   1               5                   10                  15
   Glu Gln Tyr Ile Asp Glu Ser Ile Ser Ser Ile Ile Asn Gln Thr Tyr
                   20                  25                  30
   Glu Asn Leu Glu Ile Ile Val Ile Asn Asp Gly Ser Thr Asp Leu Thr
               35                  40                  45
   Leu Ser His Leu Glu Glu Ile Ser Lys Leu Asp Lys Arg Ile Lys Ile
       50                  55                  60
   Ile Ser Asn Lys Tyr Asn Leu Gly Phe Ile Asn Ser Leu Asn Ile Gly
   65                  70                  75                  80
   Leu Gly Cys Phe Ser Gly Lys Tyr Phe Ala Arg Met Asp Ala Asp Asp
                   85                  90                  95
   Ile Ala Lys Pro Ser Trp Ile Glu Lys Ile Val Thr Tyr Leu Glu Lys
               100                 105                 110
   Asn Asp His Ile Thr Ala Met Gly Ser Tyr Leu Glu Ile Ile Val Glu
           115                 120                 125
   Lys Glu Cys Gly Ile Ile Gly Ser Gln Tyr Lys Thr Gly Asp Ile Trp
       130                 135                 140
   Lys Asn Pro Leu Leu His Asn Asp Ile Cys Glu Ala Met Leu Phe Tyr
   145                 150                 155                 160
   Asn Pro Ile His Asn Asn Thr Met Ile Met Arg Ala Asn Val Tyr Arg
                   165                 170                 175
   Glu His Lys Leu Ile Phe Asn Lys Asp Tyr Pro Tyr Ala Glu Asp Tyr
               180                 185                 190
   Lys Phe Trp Ser Glu Val Ser Arg Leu Gly Cys Leu Ala Asn Tyr Pro
           195                 200                 205
   Glu Ala Leu Val Lys Tyr Arg Leu His Gly Asn Gln Thr Ser Ser Val
       210                 215                 220
   Tyr Asn His Glu Gln Asn Glu Thr Ala Lys Lys Ile Lys Arg Glu Asn
   225                 230                 235                 240
   Ile Thr Tyr Tyr Leu Asn Lys Ile Gly Ile Asp Ile Lys Val Ile Asn
                   245                 250                 255
   Ser Val Ser Leu Leu Glu Ile Tyr His Val Asp Lys Ser Asn Lys Val
               260                 265                 270
   Leu Lys Ser Ile Leu Tyr Glu Met Tyr Met Ser Leu Asp Lys Tyr Thr
           275                 280                 285
   Ile Thr Ser Leu Leu His Phe Ile Lys Tyr His Leu Glu Leu Phe Asp
       290                 295                 300
   Leu Lys Gln Asn Leu Lys Ile Ile Lys Lys Phe Ile Arg Lys Ile Asn
   305                 310                 315                 320
   Val Ile Phe
<210> 3
   <211> 972
   <212> DNA
   <213> Haemophilus influenzae
<220>
<230> LgtD gene from Haemophilus influenzae (GenBank U32832)
<400> 3
   atggaaaatt gtccattagt atcggttatt gtttgtgctt ataacgctga gcaatatata     60
   gatgaaagca tttcatccat tattaatcag acttatgaaa atctagaaat tatagttatc    120
   aatgatggtt caacagattt gactttgtct catttagaag aaatatctaa attagataaa    180
   aggataaaaa ttatcagtaa taaatataat ttagggttca taaattcttt gaatataggc    240
   cttggttgtt tttcaggtaa atattttgca agaatggatg ctgatgatat agctaaacca    300
   tcgtggattg agaaaatagt tacctatctg gagaaaaatg atcatattac agcaatggga    360
   tcatacttag agattattgt agaaaaagaa tgtggaatta tcggttctca atataaaact    420
   ggagatatat ggaaaaatcc attgctacat aatgatattt gtgaagctat gcttttctat    480
   aatccgatac ataacaacac tatgattatg agagcaaatg tatatagaga gcataaatta    540
   atctttaata aagattatcc gtatgcagaa gattataagt tttggtcaga ggttagtagg    600
   cttggttgtt tagctaatta tcctgaagca ttagtaaaat atagactaca tggaaaccaa    660
   acatcatcag tttataatca tgagcaaaat gagacagcta aaaagataaa gagggaaaat    720
   attacatatt accttaataa gataggtata gatataaaag taattaatag tgtgtcgttg    780
   ctagaaatat atcatgtgga taaaagtaat aaagtgttga aaagtatact ttatgagatg    840
   tatatgagct tagataaata tactataact tcactcttac attttattaa atatcatctt    900
   gaattatttg atttaaagca aaatttaaag attataaaaa agttcataag aaaaataaat    960
   gttatatttt ag                                                         972
<210> 4
   <211> 323
   <212> PRT
   <213> Haemophilus influenzae
<220>
<230> LgtD protein from Haemophilus influenzae (id AAC23227)
<400> 4
   Met Glu Asn Cys Pro Leu Val Ser Val Ile Val Cys Ala Tyr Asn Ala
   1               5                   10                  15
   Glu Gln Tyr Ile Asp Glu Ser Ile Ser Ser Ile Ile Asn Gln Thr Tyr
               20                  25                  30
   Glu Asn Leu Glu Ile Ile Val Ile Asn Asp Gly Ser Thr Asp Leu Thr
           35                  40                  45
   Leu Ser His Leu Glu Glu Ile Ser Lys Leu Asp Lys Arg Ile Lys Ile
       50                  55                  60
   Ile Ser Asn Lys Tyr Asn Leu Gly Phe Ile Asn Ser Leu Asn Ile Gly
   65                  70                  75                  80
   Leu Gly Cys Phe Ser Gly Lys Tyr Phe Ala Arg Met Asp Ala Asp Asp
                   85                  90                  95
   Ile Ala Lys Pro Ser Trp Ile Glu Lys Ile Val Thr Tyr Leu Glu Lys
               100                 105                 110
   Asn Asp His Ile Thr Ala Met Gly Ser Tyr Leu Glu Ile Ile Val Glu
           115                 120                 125
   Lys Glu Cys Gly Ile Ile Gly Ser Gln Tyr Lys Thr Gly Asp Ile Trp
       130                 135                 140
   Lys Asn Pro Leu Leu His Asn Asp Ile Cys Glu Ala Met Leu Phe Tyr
   145                 150                 155                 160
   Asn Pro Ile His Asn Asn Thr Met Ile Met Arg Ala Asn Val Tyr Arg
                   165                 170                 175
   Glu His Lys Leu Ile Phe Asn Lys Asp Tyr Pro Tyr Ala Glu Asp Tyr
               180                 185                 190
   Lys Phe Trp Ser Glu Val Ser Arg Leu Gly Cys Leu Ala Asn Tyr Pro
           195                 200                 205
   Glu Ala Leu Val Lys Tyr Arg Leu His Gly Asn Gln Thr Ser Ser Val
       210                 215                 220
   Tyr Asn His Glu Gln Asn Glu Thr Ala Lys Lys Ile Lys Arg Glu Asn
   225                 230                 235                 240
   Ile Thr Tyr Tyr Leu Asn Lys Ile Gly Ile Asp Ile Lys Val Ile Asn
                   245                 250                 255
   Ser Val Ser Leu Leu Glu Ile Tyr His Val Asp Lys Ser Asn Lys Val
               260                 265                 270
   Leu Lys Ser Ile Leu Tyr Glu Met Tyr Met Ser Leu Asp Lys Tyr Thr
           275                 280                 285
   Ile Thr Ser Leu Leu His Phe Ile Lys Tyr His Leu Glu Leu Phe Asp
       290                 295                 300
   Leu Lys Gln Asn Leu Lys Ile Ile Lys Lys Phe Ile Arg Lys Ile Asn
   305                 310                 315                 320
   Val Ile Phe
<210> 5
   <211> 944
   <212> DNA
   <213> Helicobacter pylori
<220>
<230> Helicobacter pylori gene futC (GenBank AF076779)
<400> 5
   atggctttta aagtggtgca aatttgtggg gggcttggga atcaaatgtt tcaatacgct      60
   ttcgctaaaa gtttgcaaaa acaccttaat acgcccgtgc tattagacac tacttctttt     120
   gattggagca ataggaaaat gcaattagag cttttcccta ttgatttgcc ctatgcgaat     180
   gcaaaagaaa tcgctatagc taaaatgcaa catctcccca agttagtaag agatgcactc     240
   aaatacatag gatttgatag ggtgagtcaa gaaatcgttt ttgaatacga gcctaaattg     300
   ttaaagccaa gccgtttgac ttattttttt ggctatttcc aagatccacg atattttgat     360
   gctatatcct ctttaatcaa gcaaaccttc actctacccc cccccccgga aaataataaa     420
   aataataata aaaaagagga agaataccag cgcaagcttt ctttgatttt agccgctaaa     480
   aacagcgtat ttgtgcatat aagaagaggg gattatgtgg ggattggctg tcagcttggt     540
   attgattatc aaaaaaaggc gcttgagtat atggcaaagc gcgtgccaaa catggagctt     600
   tttgtgtttt gcgaagactt aaaattcacg caaaatcttg atcttggcta ccctttcacg     660
   gacatgacca ctagggataa agaagaagag gcgtattggg atatgctgct catgcaatct     720
   tgcaagcatg gcattatcgc taatagcact tatagctggt gggcggctta tttgatggaa     780
   aatccagaaa aaatcattat tggccccaaa cactggcttt ttgggcatga aaatattctt     840
   tgtaaggaat gggtgaaaat agaatcccat tttgaggtaa aatcccaaaa atataacgct     900
   taaagcggct taaaaaaagg gcttactaga ggtttaatct ttga                      944
<210> 6
   <211> 300
   <212> PRT
   <213> Helicobacter pylori
<220>
<230> Helicobacter pylori futC protein id (id AAC99764)
<400> 6
   Met Ala Phe Lys Val Val Gln Ile Cys Gly Gly Leu Gly Asn Gln Met
   1               5                   10                  15
   Phe Gln Tyr Ala Phe Ala Lys Ser Leu Gln Lys His Leu Asn Thr Pro
               20                  25                  30
   Val Leu Leu Asp Thr Thr Ser Phe Asp Trp Ser Asn Arg Lys Met Gln
           35                  40                  45
   Leu Glu Leu Phe Pro Ile Asp Leu Pro Tyr Ala Asn Ala Lys Glu Ile
       50                  55                  60
   Ala Ile Ala Lys Met Gln His Leu Pro Lys Leu Val Arg Asp Ala Leu
   65                  70                  75                  80
   Lys Tyr Ile Gly Phe Asp Arg Val Ser Gln Glu Ile Val Phe Glu Tyr
                   85                  90                  95
   Glu Pro Lys Leu Leu Lys Pro Ser Arg Leu Thr Tyr Phe Phe Gly Tyr
               100                 105                 110
   Phe Gln Asp Pro Arg Tyr Phe Asp Ala Ile Ser Ser Leu Ile Lys Gln
           115                 120                 125
   Thr Phe Thr Leu Pro Pro Pro Pro Glu Asn Asn Lys Asn Asn Asn Lys
       130                 135                 140
   Lys Glu Glu Glu Tyr Gln Arg Lys Leu Ser Leu Ile Leu Ala Ala Lys
   145                 150                 155                 160
   Asn Ser Val Phe Val His Ile Arg Arg Gly Asp Tyr Val Gly Ile Gly
                   165                 170                 175
   Cys Gln Leu Gly Ile Asp Tyr Gln Lys Lys Ala Leu Glu Tyr Met Ala
                   180                     185                     190
   Lys Arg Val Pro Asn Met Glu Leu Phe Val Phe Cys Glu Asp Leu Lys
               195                     200                     205
   Phe Thr Gln Asn Leu Asp Leu Gly Tyr Pro Phe Thr Asp Met Thr Thr
       210                     215                     220
   Arg Asp Lys Glu Glu Glu Ala Tyr Trp Asp Met Leu Leu Met Gln Ser
   225                     230                     235                     240
   Cys Lys His Gly Ile Ile Ala Asn Ser Thr Tyr Ser Trp Trp Ala Ala
                   245                     250                     255
   Tyr Leu Met Glu Asn Pro Glu Lys Ile Ile Ile Gly Pro Lys His Trp
               260                     265                     270
   Leu Phe Gly His Glu Asn Ile Leu Cys Lys Glu Trp Val Lys Ile Glu
           275                     280                     285
   Ser His Phe Glu Val Lys Ser Gln Lys Tyr Asn Ala
       290                     295                     300
<210> 7
   <211> 1116
   <212> DNA
   <213> Neisseria meningitidis
<220>
<230> Alpha-2,3-sialyltransferase of N. meningitidis (GenBank U60660)
<400> 7
   atgggcttga aaaaggcttg tttgaccgtg ttgtgtttga ttgttttttg tttcgggata      60
   ttttatacat ttgaccgggt aaatcagggg gaaaggaatg cggtttccct gctgaaggag     120
   aaacttttca atgaagaggg ggaaccggtc aatctgattt tctgttatac catattgcag     180
   atgaaggtgg cggaaaggat tatggcgcag catccgggcg agcggtttta tgtggtgctg     240
   atgtctgaaa acaggaatga aaaatacgat tattatttca atcagataaa ggataaggcg     300
   gagcgggcgt actttttcca cctgccctac ggtttgaaca aatcgtttaa tttcattccg     360
   acgatggcgg agctgaaggt aaagtcgatg ctgctgccga aagtcaagcg gatttatttg     420
   gcaagtttgg aaaaagtcag cattgccgcc tttttgagca cttacccgga tgcggaaatc     480
   aaaacctttg acgacgggac aggcaattta attcaaagca gcagctattt gggcgatgag     540
   ttttctgtaa acgggacgat caagcggaat tttgcccgga tgatgatcgg agattggagc     600
   atcgccaaaa cccgcaatgc ttccgacgag cattacacga tattcaaggg tttgaaaaac     660
   attatggacg acggccgccg caagatgact tacctgccgc tgttcgatgc gtccgaactg     720
   aagacggggg acgaaacggg cggcacggtg cggatacttt tgggttcgcc cgacaaagag     780
   atgaaggaaa tttcggaaaa ggcggcaaaa aacttcaaaa tacaatatgt cgcgccgcat     840
   ccccgccaaa cctacgggct ttccggcgta accacattaa attcgcccta tgtcatcgaa    900
   gactatattt tgcgcgagat taagaaaaac ccgcatacga ggtatgaaat ttataccttt    960
   ttcagcggcg cggcgttgac gatgaaggat tttcccaatg tgcacgttta cgcattgaaa    1020
   ccggcttccc ttccggaaga ttattggctc aagccggtgt atgccctgtt tacccaatcc    1080
   ggcatcccga ttttgacatt tgacgataaa aattaa                              1116
<210> 8
   <211> 371
   <212> PRT
   <213> Neisseria meningitidis
<220>
<230> Alpha-2,3-sialyltransferase of N. meningitidis (id AAC44541.1)
<400> 8
   Met Gly Leu Lys Lys Ala Cys Leu Thr Val Leu Cys Leu Ile Val Phe
   1               5                   10                  15
   Cys Phe Gly Ile Phe Tyr Thr Phe Asp Arg Val Asn Gln Gly Glu Arg
               20                  25                  30
   Asn Ala Val Ser Leu Leu Lys Glu Lys Leu Phe Asn Glu Glu Gly Glu
           35                  40                  45
   Pro Val Asn Leu Ile Phe Cys Tyr Thr Ile Leu Gln Met Lys Val Ala
       50                  55                  60
   Glu Arg Ile Met Ala Gln His Pro Gly Glu Arg Phe Tyr Val Val Leu
   65                  70                  75                  80
   Met Ser Glu Asn Arg Asn Glu Lys Tyr Asp Tyr Tyr Phe Asn Gln Ile
                   85                  90                  95
   Lys Asp Lys Ala Glu Arg Ala Tyr Phe Phe His Leu Pro Tyr Gly Leu
               100                 105                 110
   Asn Lys Ser Phe Asn Phe Ile Pro Thr Met Ala Glu Leu Lys Val Lys
           115                 120                 125
   Ser Met Leu Leu Pro Lys Val Lys Arg Ile Tyr Leu Ala Ser Leu Glu
       130                 135                 140
   Lys Val Ser Ile Ala Ala Phe Leu Ser Thr Tyr Pro Asp Ala Glu Ile
   145                 150                 155                 160
   Lys Thr Phe Asp Asp Gly Thr Gly Asn Leu Ile Gln Ser Ser Ser Tyr
                   165                 170                 175
   Leu Gly Asp Glu Phe Ser Val Asn Gly Thr Ile Lys Arg Asn Phe Ala
               180                 185                 190
   Arg Met Met Ile Gly Asp Trp Ser Ile Ala Lys Thr Arg Asn Ala Ser
           195                 200                 205
   Asp Glu His Tyr Thr Ile Phe Lys Gly Leu Lys Asn Ile Met Asp Asp
       210                 215                 220
   Gly Arg Arg Lys Met Thr Tyr Leu Pro Leu Phe Asp Ala Ser Glu Leu
   225                 230                 235                 240
   Lys Thr Gly Asp Glu Thr Gly Gly Thr Val Arg Ile Leu Leu Gly Ser
                   245                 250                 255
   Pro Asp Lys Glu Met Lys Glu Ile Ser Glu Lys Ala Ala Lys Asn Phe
               260                 265                 270
   Lys Ile Gln Tyr Val Ala Pro His Pro Arg Gln Thr Tyr Gly Leu Ser
           275                 280                 285
   Gly Val Thr Thr Leu Asn Ser Pro Tyr Val Ile Glu Asp Tyr Ile Leu
       290                 295                 300
   Arg Glu Ile Lys Lys Asn Pro His Thr Arg Tyr Glu Ile Tyr Thr Phe
   305                 310                 315                 320
   Phe Ser Gly Ala Ala Leu Thr Met Lys Asp Phe Pro Asn Val His Val
                   325                 330                 335
   Tyr Ala Leu Lys Pro Ala Ser Leu Pro Glu Asp Tyr Trp Leu Lys Pro
               340                 345                 350
   Val Tyr Ala Leu Phe Thr Gln Ser Gly Ile Pro Ile Leu Thr Phe Asp
           355                 360                 365
   Asp Lys Asn
       370
<210> 9
   <211> 328
   <212> PRT
   <213> Campylobacter jejuni
<220>
<230> UDP-glucose 4-epirase Campylobacter jejuni subsp. jejuni (NCTC 11168 - NCBI CAB73386.1)
<400> 9
   Met Lys Ile Leu Ile Ser Gly Gly Ala Gly Tyr Ile Gly Ser His Thr
   1               5                   10                  15
   Leu Arg Gln Phe Leu Lys Thr Asp His Glu Ile Cys Val Leu Asp Asn
               20                  25                  30
   Leu Ser Lys Gly Ser Lys Ile Ala Ile Glu Asp Leu Gln Lys Thr Arg
           35                  40                  45
   Ala Phe Lys Phe Phe Glu Gln Asp Leu Ser Asp Phe Gln Gly Val Lys
       50                  55                  60
   Ala Leu Phe Glu Arg Glu Lys Phe Asp Ala Ile Val His Phe Ala Ala
   65                  70                  75                  80
   Ser Ile Glu Val Phe Glu Ser Met Gln Asn Pro Leu Lys Tyr Tyr Met
                   85                  90                  95
   Asn Asn Thr Val Asn Thr Thr Asn Leu Ile Glu Thr Cys Leu Gln Thr
               100                 105                 110
   Gly Val Asn Lys Phe Ile Phe Ser Ser Thr Ala Ala Thr Tyr Gly Glu
           115                 120                 125
   Pro Gln Thr Pro Val Val Ser Glu Thr Ser Pro Leu Ala Pro Ile Asn
       130                 135                 140
   Pro Tyr Gly Arg Ser Lys Leu Met Ser Glu Glu Val Leu Arg Asp Ala
   145                 150                 155                 160
   Ser Met Ala Asn Pro Glu Phe Lys His Cys Ile Leu Arg Tyr Phe Asn
                   165                 170                 175
   Val Ala Gly Ala Cys Met Asp Tyr Thr Leu Gly Gln Arg Tyr Pro Lys
               180                 185                 190
   Ala Thr Leu Leu Ile Lys Val Ala Ala Glu Cys Ala Ala Gly Lys Arg
           195                 200                 205
   Asp Lys Leu Phe Ile Phe Gly Asp Asp Tyr Asp Thr Lys Asp Gly Thr
       210                 215                 220
   Cys Ile Arg Asp Phe Ile His Val Asp Asp Ile Ser Ser Ala His Leu
   225                 230                 235                 240
   Ala Ala Leu Asp Tyr Leu Lys Glu Asn Glu Ser Asn Val Phe Asn Val
                   245                 250                 255
   Gly Tyr Gly His Gly Phe Ser Val Lys Glu Val Ile Glu Ala Met Lys
               260                 265                 270
   Lys Val Ser Gly Val Asp Phe Lys Val Glu Leu Ala Pro Arg Arg Ala
           275                 280                 285
   Gly Asp Pro Ser Val Leu Ile Ser Asp Ala Ser Lys Ile Arg Asn Leu
       290                 295                 300
   Thr Ser Trp Gln Pro Lys Tyr Asp Asp Leu Glu Leu Ile Cys Lys Ser
   305                 310                 315                 320
   Ala Phe Asp Trp Glu Lys Gln Cys
                   325

## Description

### FIELD OF THE INVENTION

The present invention relates to the use of the glycosyltransferase encoded by the *lgtD* gene from Haemophilus influenzae as a β1,3 galactosyl transferase to catalyze the transfer of a galactose moiety from UDP-Gal to an acceptor bearing the terminal non reducing structure GalNAcβ-3-R to form the Galβ-3GalNAcβ-3-R structure. It also relates to the large scale in vivo synthesis of globosides oligosaccharides such as globopentaose, which are the carbohydrate portions of globotriosylceramide (Gb3Cer), globotetraosylceramide (Gb4Cer) and globopentaosylceramide (Gb5Cer) respectively and of potential anticancer vaccines of the globo-series glycosphingolipids, including the Globo-H.

### BACKGROUND OF THE INVENTION

It is now well-established that oligosaccharides play an important biological role especially as regards to the activity and function of proteins; thus, they serve to modulate the half-life of proteins, and occasionally they are involved in the structure of the protein. Oligosaccharides play an essential role in antigen variability (for example blood groups), and in certain bacterial infections such as those caused by *Neisseria meningitidis*.

As oligosaccharides are usually obtained in a low yield by purification starting from natural sources, the synthesis of oligosaccharides has become a major challenge of carbohydrate chemistry. In particular, it is a goal to supply sufficient amounts of well-characterized oligosaccharides, required for fundamental research or for any other potential applications.

The synthesis of complex oligosaccharides of biological interest may be performed chemically, enzymatically or microbiologically. Despite the development of new chemical methods for synthesizing oligosaccharides in the course of the last 20 years, the chemical synthesis of oligosaccharides remains very difficult on account of the numerous selective protection and deprotection steps, the lability of the glycoside linkages, the difficulties in obtaining regiospecific couplings, and the low production yields.

High yield production and purification of globosides has remained a challenge despite substantial utilities of these particular oligosaccharides. For example, Gb3Cer constitutes the rare P^{k} blood group antigen on erythrocytes and the CD77 differentiation antigen on lymphocytes. Gb3Cer is also the receptor for the Shiga toxins (Stx) produced by *Shigella dysenteriae* [1] and Stx-like producing *Escherichia coli* strains. Gb4Cer, known as globoside or P antigen, is the most abundant neutral glycosphingolipid in erythrocyte membranes. The P antigen has been identified as the receptor for the parvo-B19 virus [2], which was shown to bind erythroid progenitor cells via interaction with three globotetraose molecules [3]. The galabiose motif (Gala-4Gal) present in Gb3Cer, Gb4Cer and Gb5Cer is the minimal structure recognized by the PapG adhesins that are found at the tip of pili on uropathogenic *Escherichia coli* strains [4].

Globotriose has also been found in meningococcal lipooligosaccharides [5] and the *lgtC* gene encoding the α1,4 galactosyltransferase has been identified in both *Neisseria gonorrhoeae*[6] and *N*. *meningitidis* [7]. Recombinant LgtC enzyme has been successfully overproduced in *E. coli* [8] and used for large scale enzymatic synthesis of globotriose using purified enzymes [9], [10] or metabolically engineered permeabilized whole cells [11], [12]. Expression of a globotetraose epitope in a bacterial lipopolysaccharide was first reported in a capsule deficient strain of *Haemophilus influenzae* Rd [13]. The *lgtD* gene for β1,3-*N-*acetylgalactosaminyltransferase was later identified in *H*. *influenzae* Rd [14] and further characterized [10]. The enzymatic synthesis of globotetraose was reported using purified recombinant *H*. *influenzae* LgtD protein and an enzymatic UDP-GalNAc regeneration system ([15] [16]).

In addition, the globopentaose is often referred to as the stage specific embryonic antigen-3 (SSEA-3) which is the carbohydrate moiety of the galactosyl-globoside (Gb5). Gb5 is expressed by human embryonal carcinoma cells [27] and is a key intermediate for the synthesis of other tumor makers and potential anticancer vaccines of the globo-series glycosphingolipids, which include the Globo-H [28], the sialosyl galactosyl globoside [29] and the disialosyl galactosyl globoside [31]. The sialosyl galactosyl globoside was also found to be the preferred binding receptor for uropathogenic *Escherichia coli* [30] and could potentially be used as an anti-infective agent.

We have recently developed a new fermentation process for the low-cost production of lactose-derived oligosaccharides using living recombinant *E. coli* cells overexpressing the suitable glycosyltransferase genes [17]. The process is based on the active uptake of lactose while the cells are growing on an alternative substrate such as glycerol. In addition, we have designed and practiced a method which benefits from the observation that *Escherichia coli* cells are able to efficiently internalize exogenous globotriose into the cells. Here, we go further and provide a new process which can be advantageously used for the large scale production of the above mentioned globosides oligosaccharides. This process is also based on the discovery the β-3 Gal transferase activity encoded by *lgtD* is capable of efficient convertion of globotetraose into globopentaose.

### BRIEF SUMMARY OF THE INVENTION

The present invention provides methods of producing oligosaccharides by fermentative growth of microorganisms. In particular, the invention relates to a method of synthesis of "globosides" which will be understood as oligosaccharides comprising the motif Galβ-3GalNAcβ-3-R including but not limited to:
Galβ-3GalNAcβ-3Gal, Galβ-3GalNAcβ-3Galα-X, Galβ-3GalNAcβ-3Galβ-X, Galβ-3GalNAcβ-3Galβ-allyl, Galβ-3GalNAcβ-3Galβ-propragyl, Galβ-3GalNAcβ-3Galα-4Galβ-4Gal (Globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Galβ-4Galβ-allyl, and Galβ-3GalNAcβ-3Galα-4Galβ-4Galβ-propargyl;

Using in a first common step an exogenous glycosyltransferase encoded by the *lgtD* gene from Haemophilus influenzae of SEQ ID No 3 or a sequence having at least 80%, 90%, 95%, or 99% identity thereof, as a β1,3 galactosyl transferase to catalyze the transfer of a galactose moiety from UDP-Gal to an acceptor bearing the terminal non reducing structure GaNAcβ-3-R to form the Galβ-3GalNAcβ-3-R structure, wherein R is selected from the group consisting of galactose, galactose-X, β galactosides such as allyl-β-galactoside or propargyl-β-galactoside, α galactosides, globotriose, β globotrioside such as allyl-β-globotrioside or propargyl-β-globotrioside, α globotrioside, X being defined as a reactive group allowing the covalent coupling with an other molecule, including amino, azide and nitrophenyl groups.

In another embodiment, the invention relates to the use of a microorganism comprising a heterologous *lgtD* gene from Haemophilus influenzae of SEQ ID No 3 or a sequence having at least 80% identity thereof to produce an oligosaccharide selected from Galβ-3GalNAcβ-3Gal, Galβ-3GalNAcβ-3-3Galα-X, Galβ-3GalNAcβ-3Galβ-X, Galβ-3GalNAcβ-3Galβ-allyl, Galβ-3GalNAcβ-3Ga1β-propragyl, Galβ-3GalNAcβ-3Galα-4Galβ-4Gal (Globopentaose), Galβ-3GalNAcβ-3Galα-4Galβ-4Gala-X, Galβ-3GalNAcβ-3Galα-4Galβ-4Galβ-X, Galβ-3GalNAcβ-3Galα-4Gaβ-4Galβ-allyl, and Galβ-3GalNAcβ-3Galα-4Galβ-4Galβ-propargyl.
Example of produced structure R= Galβ-3GalNAcβ-3Gal Gal Galβ-3GalNAcβ-3Gala-X α galactosides, Galβ-3GalNAcβ-3Galβ-X β galactosides, Galβ-3GalNAcβ-3Galβ-allyl allyl-β-galactoside Galβ-3GalNAcβ-3Galβ-propragyl propargyl-β-galactoside Galβ-3GalNAcβ-3Galα-4Galβ-4Gal globotriose Galβ-3GalNAcβ-3Gala-4Galβ-4Galα-X α globotrioside Galβ-3GalNAcβ-3Galα-4Galβ-4Galβ-X β globotrioside Galβ-3GalNAcβ-3Galα-4Galβ-4Galβ-allyl allyl-β-globotrioside Galβ-3GalNAcβ-3Galα-4Galβ-4Galβ-propargyl propargyl-β-globotrioside

In one embodiment, **globotriose** is supplied in the culture medium and is internalized by engineered micro-organisms to produce globosides sugars such as:
**(11)** globopentaose (Galβ-3GalNAcβ-3Galα-4Galβ-4Gal),
**(12)** globo-H hexasaccharide (Fucα-2Galβ-3GalNAcβ-3Galα-4Galβ-4Gal),
**(13)** sialosyl galactosyl globoside (SGG) hexasaccharide (NeuAcα-3Galβ-3GalNAcβ-3Galα-4Galβ-4Gal), as well as
**(14)** disialosyl galactosyl globoside heptasaccharide (NeuAcα-3Galβ-3(NeuAcα-6)GalNAcβ-3Galα-4Galβ-4Gal).

This method may be extended to the production of Gb3Cer, Gb4Cer and Gb5Cer by reacting the above oligosaccharide moities with ceramide.

In another embodiment, **galactose** is supplied in the culture medium and is internalized by engineered micro-organisms and used as precursor.

In this regard, the invention contemplates a method comprising culturing a microorganism for producing :
- Galβ-3GalNAcβ-3Gal (SSEA-3 antigen)
- NeuAcα-3 Galβ-3GalNAcβ-3Gal (SSEA-4 antigen)
- Fucα-2Galβ-3GalNAcβ-3Gal (Globo-H antigen)

### DESCPRIPTION OF THE DRAWINGS

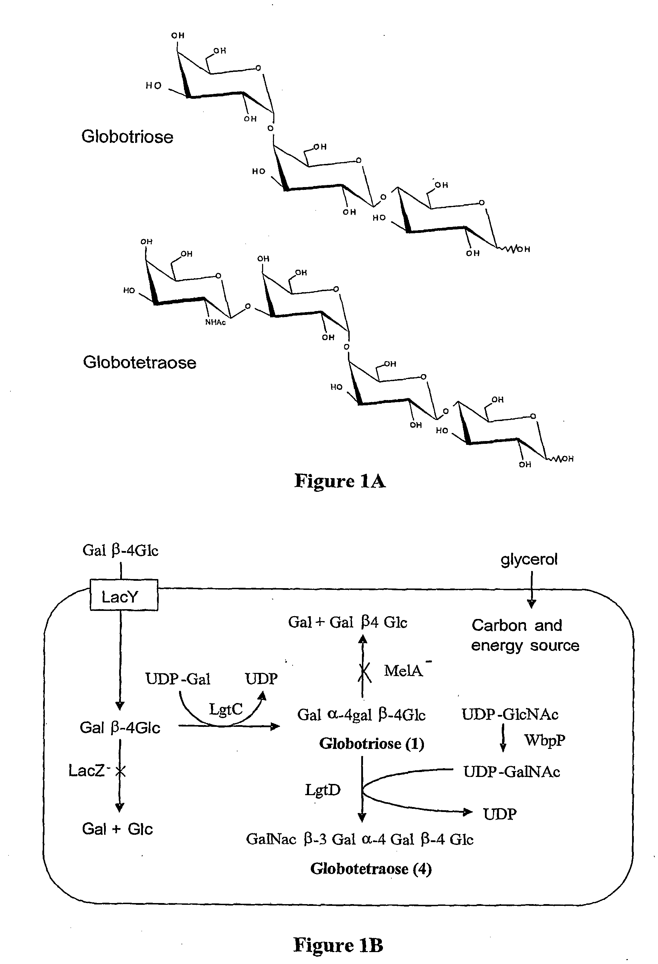

**Figure 1** shows **A)** structures of globotriose and globotetraose and **B)** a strategy for metabolically engineered pathway for globotriose **(1)** and globotetraose **(4)** production from lactose in *Escherichia coli* K 12. Lactose is internalized by the β-galactoside permease LacY and accumulates in the cytoplasm because the strain is a *lacZ* mutant devoid of β-galactosidase activity. Using the endogenous UDP-Gal pool, the α-4 galactosyltransferase (LgtC) converts lactose into globotriose (**1**). The later cannot be degraded because the *melA* gene for α-galactosidase has been inactivated. Globotriose **(1)** can be further converted to globotetraose by the β-3 GalNAc transferase encoded by *lgtD*. Since *Escherichia coli* K12 is not normally able to synthesize UDP-GalNAc, the strain was complemented with the *Pseudomonas aeruginosa wbpP* gene for UDP-GlcNAc-C4 epimerase.

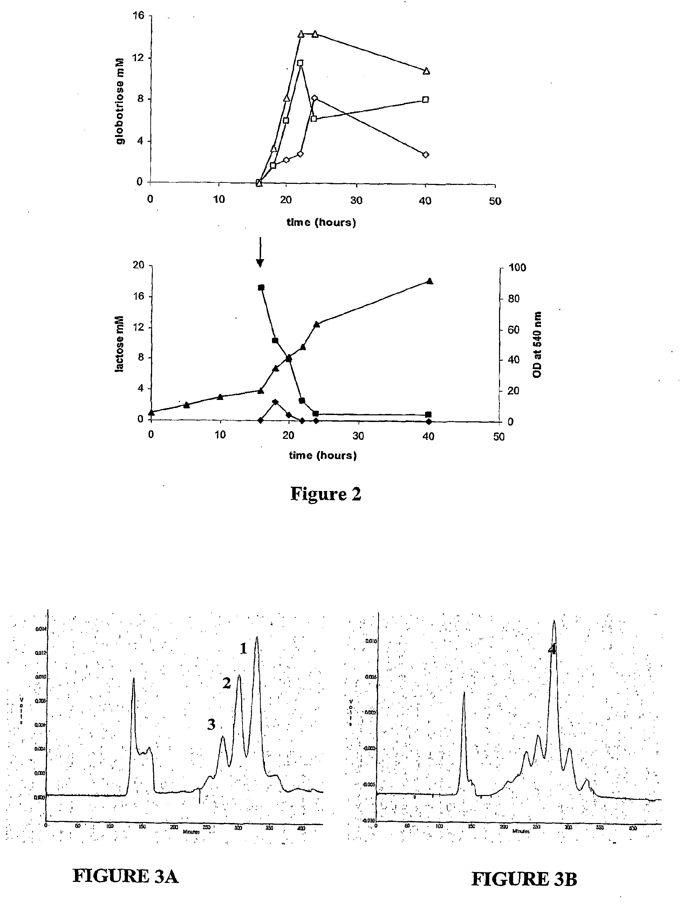

**Figure 2** shows the production of globotriose (**1**) in a high-cell density culture of the TA19 strain. (▲) bacterial growth; (■) extracellular lactose; (◆) intracellular lactose; (◇) intracellular globotriose; (□) extracellular globotriose; (Δ) sum of intracellular and extracellular globotriose. The arrow indicates the start of induction and the addition of lactose (14.6 mM).
**Figure 3** shows the separation on Biogel P2 of the intracellular oligosaccharide fraction from (A) the culture of the globotriose producing TA19 strain (compounds 1, 2 and **3**) and (B) the culture of the globotetraose-producing strain TA11 (compound **4**).

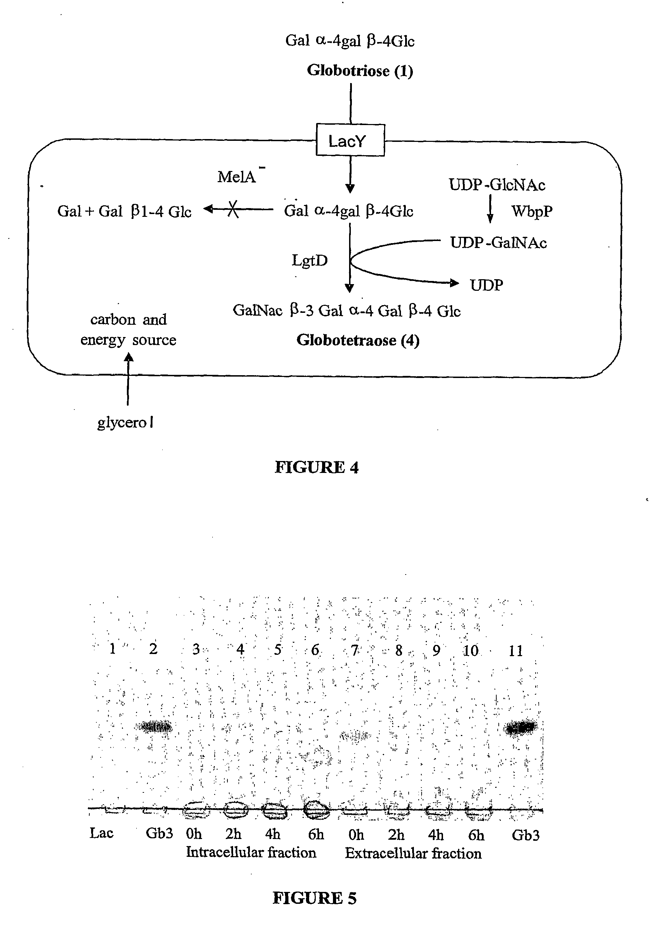

**Figure 4** displays the metabolically engineered pathway for globotetraose production from exogenous globotriose in *Escherichia coli* K 12. Globotriose (**1**) is taken up by the β-galactoside permease LacY and converted to globotetraose (**4**) by β-3 GalNAc transferase encoded by *lgtD*. Since Escherichia coli K12 is not normally able to synthesize UDP-GalNAc, the strain was complemented with the *Pseudomonas aeruginosa wbpP* gene for UDP-GlcNAc-C4 epimerase.
**Figure 5** is TLC plate analysis of the oligosaccharide content in the intracellular fraction (lanes 3, 4, 5, 6) and in the extracellular fraction (lanes 7, 8, 9, 10) of samples withdrawn from culture of the globotetraose-producing TA21 strain immediately after the addition of globotriose and after 2, 4 and 6 hours of culture, respectively. Standards are in lane 1 (lactose) and in lanes 2 and 11 (globotriose).

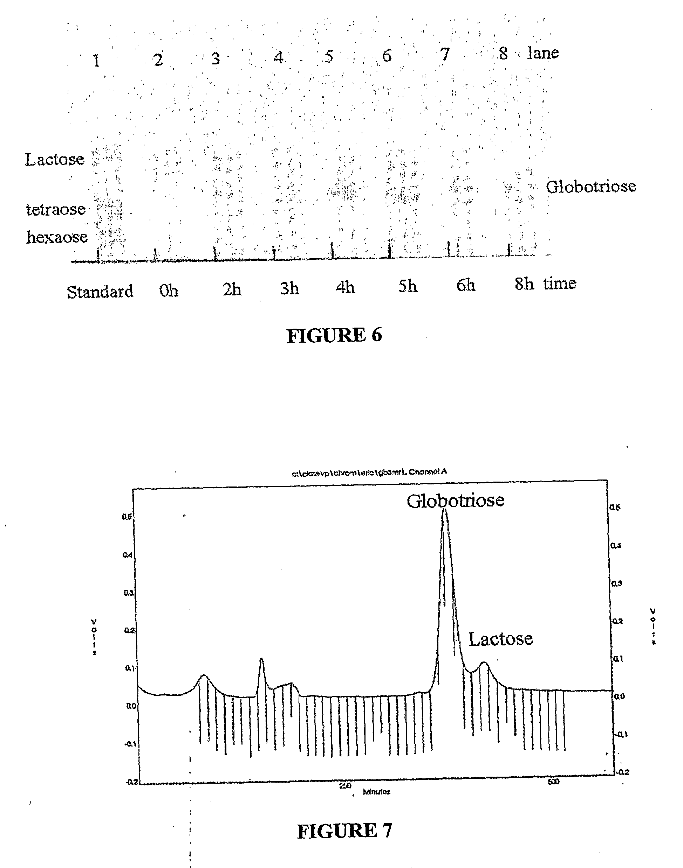

**Figure 6** shows TLC analysis of oligosaccharides produced by high cell density culture of strain TA19.The initial lactose concentration was 7.5 g.l⁻¹. Lanes 1: standard solution (2 mg.ml⁻¹ each) of lactose lacto-*N*-neotetraose (LNnT), lacto-*N-*neohexaose (LNnH). Lanes 2,3,4,5,6,7,8 : intracellular fractions withdrawn 0, 2, 3, 4 , 5, 6 and 8 hours after lactose addition.
**Figure 7** is a chromatography on Biogel P2 of the Charcoal purified fraction from the 8 hours culture of stain TA19.

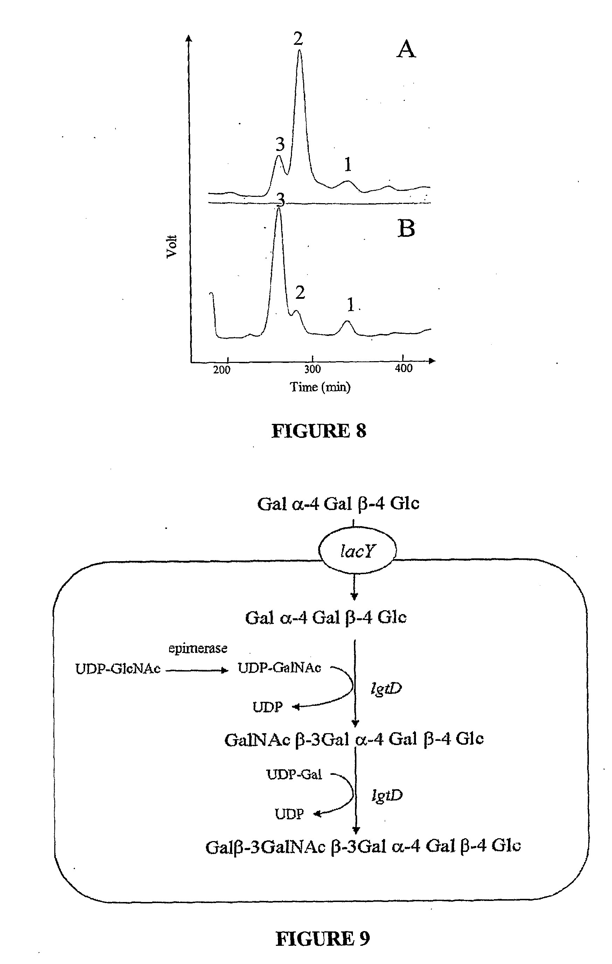

**Figure 8** is a chromatography on Biogel P2 of intracellular fraction of culture of strain TA21 harvested 6 hours (**A**) or 20 hours (**B**) after the addition of globotriose. Peak 1 and 2 and 3 were identified to globotriose, globotetraose and globopentaose respectively.
**Figure 9** displays the metabolically engineered pathway for globopentaose production from exogenous globotriose in *Escherichia coli K 12.* Globotriose is taken up by the β-galactoside permease LacY and converted into globotetraose by the β-3 GalNAc transferase encoded by *lgtD*. Since *Escherichia coli K12* is not normally able to synthesize UDP-GalNAc, the strain was complemented with the Pseudomonas aeruginosa *wbpP* gene for UDP-GlcNAc-C4 epimerase. The globotetraose is then converted into globopentaose by the β-3 Gal transferase activity encoded by *lgtD*.

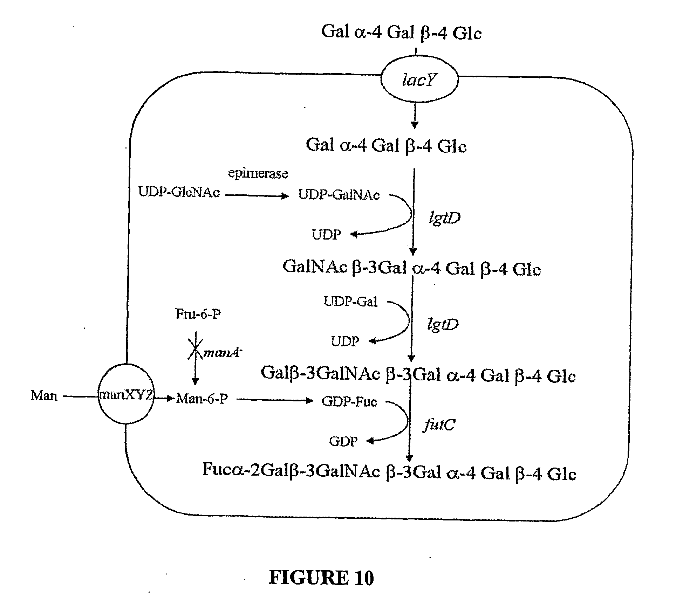

**Figure 10** displays the metabolically engineered pathway for the production of the Globo-H hexasaccharide (Fucα-2Galβ-3GalNAcβ-3Galα-4Galβ-4Gal) from exogenous globotriose in *Escherichia coli K 12*. Globotriose is taken up by the β-galactoside permease LacY and converted into globotetraose by the β-3 GalNAc transferase encoded by *lgtD*. Since *Escherichia coli K12* is not normally able to synthesize UDP-GalNAc, the strain was complemented with the Pseudomonas aeruginosa *wbpP* gene for UDP-GlcNAc-C4 epimerase. The globotetraose is then converted into globopentaose by the β-3 Gal transferase activity encoded by *lgtD*. The globopentaose is finally converted into the Globo-H hexasaccharide by the α-2 fucosyltranferase encoded by *futC*.

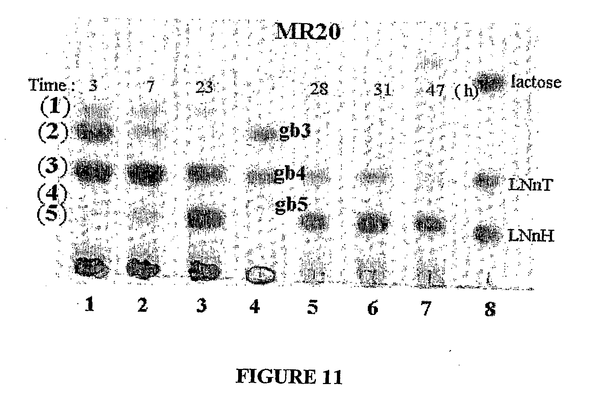

**Fig. 11****.** TLC analysis of oligosaccharides produced by high cell density culture of strain MR20. The initial globotriose concentration was 3 g.l⁻¹. Lane 4 : standard solution of Globotriose, Globotetraose and Globopentaose. Lanes 8: standard solution (2 mg.ml⁻¹ each) of lactose, lacto-*N*-neotetraose (LNnT) lacto-N-neohexaose (LNnH). Lanes 1, 2, 3, 5, 6, and 7: intracellular fractions withdrawn 3, 7, 23, 28, 31 and 47 hours after globotriose addition addition.

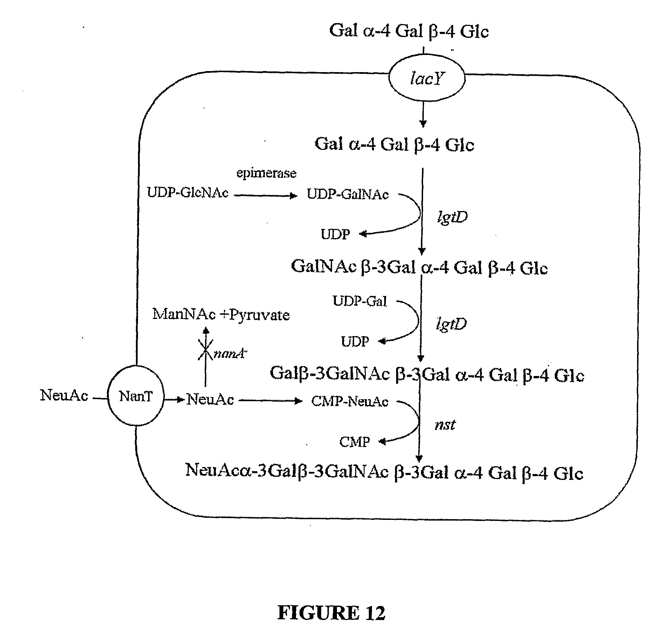

**Figure 12** shows the metabolically engineered pathway for the production of the SGG hexasaccharide (NeuAcα-3Galβ-3GalNAcβ-3Gala-4Galβ-4Gal) from exogenous globotriose and NeuAc in *Escherichia coli K 12*. Globotriose is taken up by the β-galactoside permease LacY and converted into globotetraose by the β-3 GalNAc transferase encoded by *lgtD*. Since *Escherichia coli K12* is not normally able to synthesize UDP-GalNAc, the strain was complemented with the Pseudomonas aeruginosa *wbpP* gene for UDP-GlcNAc-C4 epimerase. The globotetraose is then converted into globopentaose by the β-3 Gal transferase activity encoded by *lgtD*. The globopentaose is finally converted into the SGG hexasaccharide by the Neisseria α-3 sialyltansferase. CMP-NeuAc is produced from exogenous NeuAc which is taken up by the NanT permease. To prevent the catabolism of NeuAc, a mutant *nanA*⁻ strain devoid ofNeuAc aldolase activity is used.

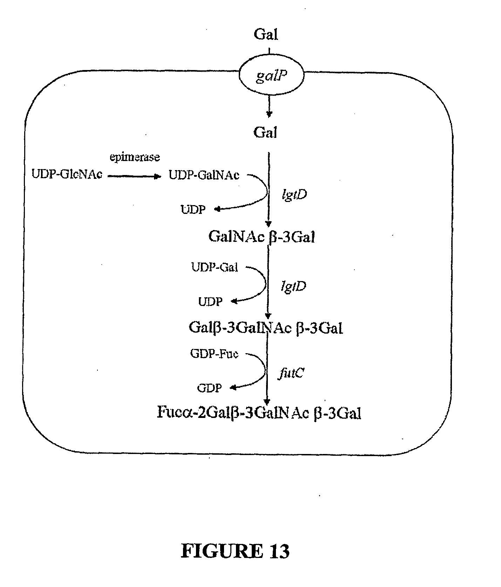

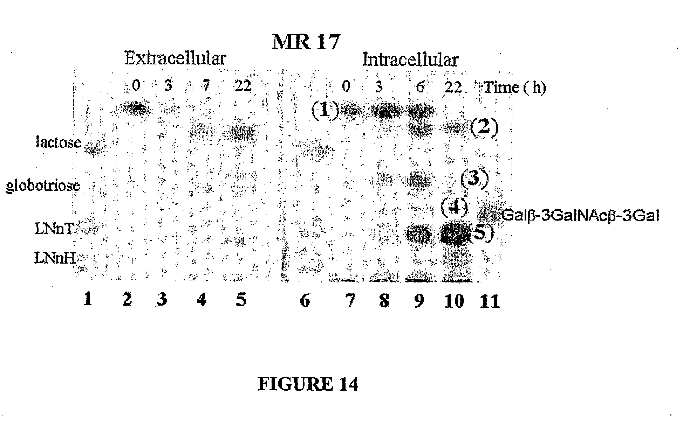

**Fig. 13** Use of galactose as acceptor for the production of the terminal structure of globo-H. The strain MR17 was constructed by transforming the GLK host strain with the three plasmids pBS-lgtD-gne, pBBRGAB and pWKS-lgtC to produce the terminal tetrasaccharide epitope of the Globo-H antigen as illustrated in **Fig 13**. Culture of strain MR17 in presence of 3 g.l⁻¹ of galactose led to the formation of major compound (**5**) that migrated in TLC as a tetrasaccharide (**Fig. 14**). Compound (**5**) was purified by adsorption on activated charcoal and size exclusion chromatography with a yield of 1.66 gram starting from a one liter culture. Identification of (**5**) as Fucα-2Galβ-3GalNAcβ-3Gal was confirmed by mass spectrometry and NMR analysis. Significant amount of a compound (2) that migrated slower than galactose (**1**) but faster than lactose was recovered in both the intra and extra cellular fraction and was identified as the disaccharide Fucα-2Gal. Two other compounds (**3**) and (**4**) transiently accumulated and were identified to the disaccharide GalNAcβ-3Gal and the trisaccharide Galβ-3GalNAcβ-3Gal by their migration rate in TLC and their mass spectrometry data.
**Fig. 14****.** TLC analysis of oligosaccharides produced by high cell density culture of strain MR17 (GLK, pBS-lgtD-gne, pBBR-GAB, pWKS-futC). The initial galactose concentration was 3 g.l⁻¹. Lanes 1 and 6 : standard solution (2 mg.ml⁻¹ each) of lactose, lacto-*N*-neotetraose (LNnT) lacto-*N*-neohexaose (LNnH) and globotriose .Lane 11 standard solution of the trisaccharide Galβ-3GalNAcβ-3Gal. Lanes 2, 3, 4, and 5: extracellular fractions withdrawn 0, 3,7, and 22 hours after galactose addition. Lanes 7, 8, 9 and 10: intracellular fractions withdrawn 0, 3, 7, and 22 hours after galactose addition.

### DETAILED DESCRIPTION OF THE INVENTION

In a specific embodiment, and referring to the above, the invention is aimed at the use of a microorganism which is *LacY*+*, LacZ*-, *melA*- and comprises a heterologous *lgtD* gene from Haemophilus influenzae of SEQ ID No 3 or a sequence having at least 80%, 90%, 95%, or 99% identity thereof and encoding β-3 GalNAc transferase, and a *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C*. *jejuni* strain NCTC 11168 of SEQ ID No 9 for producing Galβ-3GalNAcβ-3Galα-4Galβ-4Gal (Globopentaose).

In this regards, it is possible to practice the invention starting directly with globotriose in the culture medium. Alternatively, it is possible to start with lactose as precursor to produce internally globotriose, which is processed further internally to lead to globopentaose.

### Method for Globopentaose production starting from globotriose in the culture medium

The method for producing Galβ-3GalNAcβ-3Galα-4Galβ-4Gal (Globopentaose), comprises the step of a culturing a microorganism in a culture medium comprising globotriose (Galα-4Galβ-4Glc), wherein said microorganism comprises a heterologous a *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C*. *jejuni* strain NCTC 11168 of SEQ ID No 9 and a heterologous *lgtD* gene from Haemophilus influenzae of SEQ ID No 3 or a sequence having at least 80%, 90%, 95%, or 99% identity thereof and encoding β-3 GalNAc transferase. The *lgtD* gene transfers a GalNAc moiety from UDP-GalNAc to globotriose to form globotetraose (GalNAcβ-3Galα-4Galβ-4Glc) as well as a galactose moiety from UDP-Gal to globotetraose to form globopentaose (Galβ-3GalNAcβ-3Galα-4Galβ-4Gal).

This particular embodiment is illustrated in **Fig. 9**.

### Globopentaose production starting from lactose in a one-step fermentation process

In one particular embodiment, the invention is directed to a method comprising culturing a microorganism in a culture medium comprising excess lactose, wherein said microorganism comprises a heterologous gene encoding α-1,4-Gal transferase (*e*.*g*., a *lgtC* gene) which transfers a galactose moiety from UDP-Gal to the lactose to form globotriose (Galα-4Galβ-4Glc) and a heterologous *lgtD* gene from Haemophilus influenzae of SEQ ID No 3 or a sequence having at least 80%, 90%, 95%, or 99% identity thereof and encoding β-3 GalNAc transferase, which transfers a GalNAc moiety from UDP-GalNAc to the globotriose to form globotetraose (GalNAcβ-3Galα-4Gal-4Glc) as well as a galactose moiety from UDP-Gal to globotetraose to form globopentaose (Galβ-3GalNAcβ-3Galα-4Galβ-4Gal). Advantageously, this microorganism also comprises a gene encoding for UDP-GlcNAc-C4 epimerase, such as the *Pseudomonas aeruginosa wbpP* gene, or a *gne* gene encoding for a UDP-glucose 4-epimerase, such as the *gne* gene of C. *jejuni* strain NCTC 11168 of SEQ ID No 9. In addition, this microorganism is advantageously LacY+ (β-galactoside permease) and LacZ- as well as melA-, meaning that the *melA* gene for α-galactosidase activity (melibiase) had been inactivated [21]. In a particular embodiment, the micoorganism is an *E. coli* K12 strain JM107 derivative (ATCC 47014). This particular method is basically as shown in **Fig. 1B** **and** **9**. In some embodiments, the invention also relates to a microorganism, for example an *E. coli,* comprising a heterologous *lgtC* gene encoding α-1,4-Gal transferase and a heterologous *lgtD* gene from Haemophilus influenzae of SEQ ID No 3 or a sequence having at least 80%, 90%, 95%, or 99% identity thereof encoding β-3 GalNAc transferase and which is *LacY*+ (β-galactoside permease), *LacZ*- (β galactosidase), and *MelA*- (α-galactosidase). This microorganism comprises advantageously a *wbpP* gene encoding for UDP-GlcNAc-C4 epimerase or a *gne* gene encoding for a UDP-glucose 4-epimerase, such as the *gne* gene of C. *jejuni* strain NCTC 11168 of SEQ ID No 9.

### Globopentaose production coupled with Globotriose production - a two-step fermentation process

In another specific embodiment, the method may include a prelimary step comprising the high scale production of globotriose using a (first) microorganism in a culture medium comprising lactose, wherein said (first) microorganism comprises a heterologous gene encoding α-1,4-Gal transferase (*e*.*g*., a lgtC gene) which transfers a galactose moiety from UDP-Gal to the lactose to form globotriose (Galα-4Galβ-4Glc) and wherein lactose is in excess in the culture medium.

Indeed, we show hereafter that globotriose (**1**) can be produced in high yields (7 g.l⁻¹) by bacterial fermentation. However, characterization of the oligosaccharide fraction revealed that a series of galactosylated derivatives of globotriose may also be synthesized at the end of the fermentation time course. With excess lactose in the culture, however, we didn't observe any polygalactosylation until the complete consumption of lactose had been reached. Thus, polygalactosylation can be avoided by terminating the culture before the exhaustion of lactose. Furthermore, our conditions allow the recovery of most of the globotriose in the extracellular medium, because the presence of lactose prevents the re-entry of globotriose which has rapidly diffused outside the cells. Thus, in the method described above the α-1,4-Gal transferase enzyme can be encoded for example by *LgtC* genes of *N*. *meningitidis, N. gonorrhoeae* or *Haemophilus influenzae*, more particularly by the *LgtC* gene of *Neisseria meningitidis* L1 (126E) GenBank accession number U65788 - SEQ ID No 1, protein_id AAB48385 - SEQ ID No 2). One of skill will recognize that enzymes that are encoded by nucleic acids that are at least substantially identical (as defined below) to the exemplified sequences can also be used. The method as depicted above may include terminating the culture before the exhaustion of lactose and extracting globotriose molecules from the extracellular medium. Besides, increasing the concentration to reach from 5 g.L-1 to 10 g.L-1 lactose, preferably about 7.5 g.L-1 prevent the formation of polygalactosylated side-products such as polygalactosylated tetrasaccharide (Galα-4Galα-4Galβ-4Gal) and pentasaccharide. (Galα-4Galα-4Galα-4Galβ-4Gal). This results in significant improvement of globotriose yield. Advantageously, the culture can be terminated at about 8 hours after lactose addition to prevent a further galactosylation of globotriose. Optionally, this method may further include a separation and/or purifying step to recover globotriose molecules from the medium. A concentration or lyophilization step may also be included to prepare a globotriose composition such as a solution or a dry product suitable for different utilities or as a commodity. As explained before, purification steps allow one to prepare such solution or dry product with high globotriose purity, such as more than 80%, 85%, 90%, 95% or even 99% globotriose by weight of the total composition. Preferably, the method comprises terminating the culture before the exhaustion of lactose, extracting globotriose molecules in the extracellular medium and providing said globotriose molecules as a precursor The above produced globotriose may be added to a second metabolically engineered micororganism to produce globopentaose and other "globosides" oligosaccharides. The invention contemplates a method for producing globopentaose via globotetraose; comprising culturing a said second microorganism in a culture medium comprising globotriose, wherein said second microorganism comprises a heterologous gene from Haemophilus influenzae of SEQ ID No 3 or a sequence having at least 80%, 90%, 95%, or 99% identity thereof encoding β-3 GalNAc transferase as depicted above which transfers a GalNAc moiety from UDP-GalNAc to globotriose to form globotetraose (GalNAcβ-3Galα-4Galβ-4Glc) as well as a galactose moiety from UDP-Gal to globotetraose to form globopentaose (Galβ-3GalNAcβ-3Galo-4Galβ-4Gal). Advantageously, said second microorganism also comprises a gene encoding for UDP-GlcNAc-C4 epimerase, such as the *Pseudomonas aeruginosa wbpP* gene or a *gne* gene encoding for a UDP-glucose 4-epimerase, such as the *gne* gene of *C*. *jejuni* strain NCTC 11168 of SEQ ID No 9. This particular embodiment is illustrated in **Fig. 4** **and** **9**. In addition, both first and second microorganisms are typically engineered to enhance the efficiency of the claimed methods. Thus, the microorganisms preferrably encode a protein that facilitates uptake of lactose and lack enzymes that metabolize lactose. For example in *E. coli,* the cell is advantageously *LacY*+ (β-galactoside permease) and *LacZ*-. Besides, these microorganisms are preferably engineered to lack α-galactosidase activity. In *E. coli,* the cell is typically *melA*-, meaning that the *melA* gene for α-galactosidase activity (melibiase) had been inactivated [21]. In a particular embodiment, the micoorganism is an *E. coli* K12 strain JM107 derivative (ATCC 47014). It is also preferred to add glycerol in the medium as carbon and energy source.

Here, the invention also contemplates in some embodiments a set of two separate microorganisms, the first microorganism being *LacY*+, *LacZ*-, and *MelA*- and comprising a heterologous *lgtC* gene; the second being *LacY*+, *MelA*- and comprising a heterologous *lgtD* gene from Haemophilus influenzae of SEQ ID No 3 or a sequence having at least 80%, 90%, 95%, or 99% identity thereof encoding β-3 GalNAc transferase, and a heterologous *wbpP* gene or a *gne* gene encoding for a UDP-glucose 4-epimerase, such as the *gne* gene of *C*. *jejuni* strain NCTC 11168 of SEQ ID No 9.

This method coupling first the production of globotriose with said first microorganism as defined above, then producing globotetraose and other globosides using said second microorganism as mentioned above is possible because globotriose is in fact internalized by an active process which is probably be mediated by, for example, a β-galactoside permease expressed in our system. β-galactoside permease has a broad substrate specificity and has been shown to internalize α-galactosides such as the trisaccharide raffinose [24]. However it is also possible that Globotriose is internalized by other E; coli sugar permease such as the melibiose transporter encoded by the *melB* gene. Nevertheless, globotriose internalization came as a surprise since we had already observed that short oligosaccharides such as the trisaccharide LNT2 diffused rapidly in the extracellular medium [17] and that no re-entry into the cells was observed. Thus, depending on the endpoint, a convenient way to avoid the production of multiple side-products during globotetraose synthesis is to use globotriose as the acceptor instead of lactose, as described in the culture of the TA21 strain (see examples below). Besides, increasing the concentration to reach from 5 g.L-1 to 10 g.L-1 lactose, preferably about 7.5 g.L-1 prevent the formation of polygalactosylated side-products such as polygalactosylated tetrasaccharide (Galα-4Galα-4Galβ-4Gal) and pentasaccharide (Galα-4Galα-4Galα-4Galβ-4Gal). This results in significant improvement of globotriose yield. Advantageously, the culture can be terminated at about 8 hours after lactose addition to prevent a further galactosylation of globotriose.

### Globo-H production

In still another embodiment, the invention is aimed at a method for producing Globo-H hexasaccharide **(12)** comprising culturing a microorganism in a culture medium comprising globotriose, wherein said microorganism comprises a heterologous *lgtD* gene from Haemophilus influenzae of SEQ ID No 3 or a sequence having at least 80%, 90%, 95%, or 99% identity thereof encoding β-3 GalNAc transferase which transfers a GalNAc moiety from UDP-GalNAc to globotriose to form globotetraose (GalNAcβ-3Galα-4Galβ-4Glc), extending the culture to allow said β-3 GalNAc transferase to transfer a galactose moiety from UDP-Gal to globotetraose to form globopentaose (Galβ-3GalNAcβ-3Galα-4Galβ-4Gal) and wherein said microorganism further comprises a heterologous gene encoding an α-2 fucosyltranferase (*e*.*g*., a *futC* gene) to transfer a fucose moiety from GDP-Fuc to globopentaose to form Globo-H hexasaccharide (Fucα-2Galβ-3GalNAcβ-3Galα-4Galβ-4Gal). Advantageously, this microorganism also comprises a gene encoding for UDP-GlcNAc-C4 epimerase, such as the *Pseudomonas aeruginosa wbpP* gene or a *gne* gene encoding for a UDP-glucose 4-epimerase, such as the *gne* gene of *C*. *jejuni* strain NCTC 11168 of SEQ ID No 9. In one particular embodiment, the strain may be a mutant engineered so that it is unable to synthesize GDP-Fuc, unless mannose is exogenously added. For example, an *E. coli* which is *manA*⁻ devoid of phophomannose isomerase activity and *manXYZ*+. Indeed, this mutant will be unable to synthesize GDP-Fuc, unless mannose is exogenously added in the medium and taken up and phosphorylated in Man-6-P by the mannose permease encoded by the *manXYZ* genes. By adding the mannose after the entire conversion of globotriose into globopentaose, the fucosylation of globotriose will be impossible and all the globotriose will be converted into Globo-H oligosaccharide. This particular embodiment is illustrated in **Fig. 10**. In another embodiment, since we observed that fucosyltransferase is not very active on globotriose, this strain may simply be *LacY*+, *MelA*-. In this regard, the invention contemplates said second microorganism which is *LacY*+, *manA*⁻ and which comprises a heterologous a *lgtD* gene from Haemophilus influenzae of SEQ ID No 3 or a sequence having at least 80%, 90%, 95%, or 99% identity thereof (β-3 GalNAc transferase), a heterologous *wbpP* gene (UDP-GlcNAc-C4 epimerase), such as the *Pseudomonas aeruginosa wbpP* gene or a *gne* gene encoding for a UDP-glucose 4-epimerase, such as the *gne* gene of *C. jejuni* strain NCTC 11168 of SEQ ID No 9 and a heterologous *futC* gene (α-2 fucosyltranferase), such as the *Helicobacter pylori* gene *futC* of SEQ ID No 5. Thus, the *MelA*- and *manXXZ*+ features arre optional as well as the addition of mannose.

The invention also relates to a microorganism, which in some embodiments is *LacY*+, *MelA*-, *manXYZ*+, and optionally *manA*⁻ ; comprising, for example, a heterologous *lgtD* gene from Haemophilus influenzae of SEQ ID No 3 or a sequence having at least 80%, 90%, 95%, or 99% identity thereof (β-3 GalNAc transferase), a heterologous *wbpP* gene (UDP-GlcNAc-C4 epimerase), such as the *Pseudomonas aeruginosa wbpP* gene or a *gne* gene encoding for a UDP-glucose 4-epimerase, such as the *gne* gene of *C*. *jejuni* strain NCTC 11168 of SEQ ID No 9 and a heterologous *futC* gene (α-2 fucosyltranferase), such as the *Helicobacter pylori* gene *futC* from strain UA802 (GenBank accession number AF076779 - SEQ ID No 5, protein_id=AAC99764 - SEQ ID No 6) or from strain 26695 (HP0094 and HP0093, GenBank AE000531) to a cell culture medium comprising the above microorganism, globotriose (for example 1 to 10 g.L⁻¹) and mannose (for example 0.35 to 3.5 g.L⁻¹). One of skill will recognize that enzymes that are encoded by nucleic acids that are at least substantially identical (as defined below) to the exemplified sequences can also be used.

Here, the invention also contemplates a set of two separate micoorganisms, the first microorganism being *LacY*+, *LacZ*-, and *MelA*- and comprising a heterologous *lgtC* gene; the second being *LacY*+, *MelA*-, *manXXZ*+, *manA*⁻ and comprising heterologous *lgtD* gene from Haemophilus influenzae of SEQ ID No 3 or a sequence having at least 80%, 90%, 95%, or 99% identity thereof, *wbpP (or gne),* and *futC* genes.

### Sialosyl galactosyl globoside production

In still another embodiment, the invention is aimed at a method for producing sialosyl galactosyl globoside (SGG) hexasaccharide **(13)** comprising culturing a microorganism in a culture medium comprising globotriose, wherein said microorganism comprises a heterologous *lgtD* gene from Haemophilus influenzae of SEQ ID No 3 or a sequence having at least 80%, 90%, 95%, or 99% identity thereof encoding β-3 GalNAc transferase which transfers a GalNAc moiety from UDP-GalNAc to the globotriose to form globotetraose (GalNAcβ-3Galα-4Galβ-4Glc), extending the culture to allow said β-3 GalNAc transferase to transfer a galactose moiety from UDP-Gal to the globotetraose to form globopentaose (Galβ-3GalNAcβ-3Galα-4Galβ-4Gal) and wherein said microorganism comprises a gene for the CMP-NeuAc synthase (for example from *N*. *meningitidis*) and a heterologous gene encoding α-3 sialyltransferase (for example from *N*. *meningitidis*, such the MC58 strain : GenBanK accession number U60660 - SEQ ID No 7, protein_id=AAC44541.1 - SEQ ID No 8) catalyzes the transfer of a sialyl moiety from an activated sialic acid molecule to globopentaose to form sialosyl galactosyl globoside (SGG) hexasaccharide (NeuAcα-3Galβ-3GalNAcβ-3Galα-4Galβ-4Gal). One of skill will recognize that enzymes that are encoded by nucleic acids that are at least substantially identical (as defined below) to the exemplified sequences can also be used. Advantageously, this microorganism also comprises a *wbpP* gene encoding for UDP-GlcNAc-C4 epimerase, such as the *Pseudomonas aeruginosa wbpP* gene or a *gne* gene encoding for a UDP-glucose 4-epimerase, such as the *gne* gene of C. *jejuni* strain NCTC 11168 of SEQ ID No 9. It is also advantageous that the strain is a mutant nanA- so as to be devoid of Neu5Ac aldolase activity and *NanT*+ allowing active transport of sialic acid. This particular embodiment is illustrated in **Fig. 11**.

The invention also relates to a microorganism, which in preferred embodiments is *LacY*+, *MelA*-, *nanT*+, *nanA*⁻ comprising, for example, a heterologous a *lgtD* gene from Haemophilus influenzae of SEQ ID No 3 or a sequence having at least 80%, 90%, 95%, or 99% identity thereof (β-3 GalNAc transferase), a heterologous *wbpP* gene (UDP-GlcNAc-C4 epimerase), such as the *Pseudomonas aeruginosa wbpP* gene or a *gne* gene encoding for a UDP-glucose 4-epimerase, such as the *gne* gene of C. *jejuni* strain NCTC 11168 of SEQ ID No 9 and a heterologous gene for α-3 sialyltransferase, such as the gene from *N*. *meningitis* strain MC58 It also relates to a cell culture medium comprising the above microorganism, globotriose (for example 1 to 10 g.L⁻¹) and sialic acid (for example from 0.6 to 6 g.L⁻¹).
Here, the invention also contemplates a set of two separate micoorganisms, the said first microorganism being as mentioned above *LacY*+, *LacZ*-, and *MelA*- and comprising a heterologous *lgtC* gene; the second being *LacY*+, *MelA*-, *nanT*+, *nanA*⁻ and comprising heterologous *lgtD* gene from Haemophilus influenzae of SEQ ID No 3 or a sequence having at least 80%, 90%, 95%, or 99% identity thereof, *wbpP* (or *gne*) and *nst* genes.

It will be understood herein that the extracellular globotriose source used in the above methods for producing globosides such as globotetraose, globopentaose, globo-H, and sialosyl galactosyl globoside (SGG) hexasaccharide may originate from culturing said first microorganism as described above (coupling system) which produces globotriose or in still antoher embodiment from any other source (non-coupling system). Thus, is embraced by the invention the method described above for producing globotetraose, globopentaose, globo-H, sialosyl galactosyl globoside (SGG) hexasaccharide wherein it comprises a first step using the said first microorganism which is *LacY*+, *LacZ*-, and *MelA-* and which comprises a heterologous *lgtC* gene. In other words, a set of microorganisms can be used. Alternatively, it is also within the invention to directly provide globotriose from any other source to the culture medium of said second micoorganism. In this regard, the invention encompasses a method for producing an oligosaccharide comprising the galabiose motif (Galα-4Gal), referred as globosides, selected the group consisting of globotetraose, globopentaose, and galactosyl-globosides including globo-H hexasaccharide, sialosyl galactosyl globoside (SGG) hexasaccharide, disialosyl galactosyl globoside comprising the step consisting of culturing a said second microorganism as defined above in a medium comprising globotriose. The invention also concerns a culture medium comprising globotriose preferably at a concentration ranging from 1 to 10 g.L⁻¹ and the use of the said first micoorganism and methods thereof to prepare a culture medium comprising globotriose. The present disclosure also relates to the production of commercial scale compositions of the above globosides, such as a composition comprising one or several globoside(s) selected from the group consisting of globotriose, globotetraose, globopentaose, and galactosyl-globosides including globo-H hexasaccharide, and sialosyl galactosyl globoside (SGG) hexasaccharide.

### Method of production starting from galactose in the culture medium

In still another embodiment, the invention relates to the use of a *lgtD* gene from Haemophilus influenzae of SEQ ID No 3 or a sequence having at least 80%, 90%, 95%, or 99% identity thereof encoding a GalNAc transferase to transfer a GalNAc residue to galactose to form the intermediate GalNAcβ-3Gal and to produce oligosacharrides comprising GalNAcβ-3Gal. It also relates to the use of a *lgtD* gene from Haemophilus influenzae of SEQ ID No 3 or a sequence having at least 80%, 90%, 95%, or 99% identity thereof encoding a GalNAc transferase as a Gal transferase in presence of GalNAcβ-3Gal to form the terminal trisaccharide structure of the SSEA-3 antigen (Galβ-3GalNAcβ-3Gal). It also relates to a method for producing an oligosaccharide comprising the motif GalNAcβ-3Gal, the method comprising culturing a microorganism which is *galP* (galactose permease), *LacZ* (β galactosidase), *MelA-*(α-galactosidase) and *wbpP* encoding for UDP-GlcNAc-C4 epimerase, such as the *Pseudomonas aeruginosa wbpP* gene ; or a *gne* gene encoding for a UDP-glucose 4-epimerase, such as the *gne* gene of C. *jejuni* strain NCTC 11168 of SEQ ID No 9, in a culture medium comprising galactose, wherein said microorganism comprises a heterologous *lgtD* gene from Haemophilus influenzae of SEQ ID No 3 or a sequence having at least 80%, 90%, 95%, or 99% identity thereof encoding α-1,4-Gal transferase which transfers a GalNAc residue to galactose to form the an oligosaccharide comprising GalNAcβ-3Gal. In this method, the *lgtD* gene can be allowed to further transfer a galactose moiety from UDP-Gal to GalNAcβ-3Gal to form the terminal trisaccharide structure of the SSEA-3 antigen (Galβ-3GalNAcβ-3Gal). This method can be extended to the production of the terminal tetrasaccharide structure of the SSEA-4 antigen (NeuAcα-3Galβ-3GalNAcβ-3Gal). In this regard, the microorganism further comprises a heterologous gene encoding an α-3 -sialyltransferase to transfer a sialic acid moiety from CMP-NeuAc to Galβ-3GalNAcβ-3Gal to form NeuAcα-3Galβ-3GalNAcβ-3Gal. This method can be extended to the production of the terminal tetrasaccharide structure of the Globo-H antigen (Fucα-2Galβ-3GalNAcβ-3Gal). In this regard, the microorganism further comprises a heterologous *futC* gene encoding an α-2 fucosyltranferase to transfer a sialic acid moiety from GDP-Fuc to Galβ-3GalNAcβ-3Gal to form Fucα-2Galβ-3GalNAcβ-3Gal.

The invention contemplates the above microorganism as well as a culture medium comprising galactose and said microorganism.

The nomenclature and general laboratory procedures required to practice the present invention are well known to those of skill in the art. These procedures can be found, for example, in Sambrook, et al., Molecular Cloning: A Laboratory Manual (2nd Ed.), Vol. 1-3, Cold Spring Harbor Laboratory, Cold Spring Harbor, New York, 1989.

### DEFINITIONS

An "acceptor substrate" or an "acceptor saccharide" for a glycosyltransferase is an oligosaccharide moiety that can act as an acceptor for a particular glycosyltransferase. When the acceptor substrate is contacted with the corresponding glycosyltransferase and sugar donor substrate, and other necessary reaction mixture components, and the reaction mixture is incubated for a sufficient period of time, the glycosyltransferase transfers sugar residues from the sugar donor substrate to the acceptor substrate. For example, an acceptor substrate for the production of globotetraose, globopentaose, globo-H, sialosyl galactosyl globoside (SGG) hexasaccharide and disialosyl galactosyl globoside using heterologous *lgtD*, *futC* and/or *nst* in the methods of the invention is globotriose.

A "donor substrate" for glycosyltransferases is an activated nucleotide sugar. Such activated sugars generally consist of uridine, guanosine, and cytidine monophosphate derivatives of the sugars (UMP, GMP and CMP, respectively) or diphosphate derivatives of the sugars (UDP, GDP and CDP, respectively) in which the nucleoside monophosphate or diphosphate serves as a leaving group. For example, a donor substrate for α-2 fucosyltranferase used in the methods of the invention is GDP-Fuc.

A "culture medium" refers to any liquid, semi-solid or solid media that can be used to support the growth of a microorganism used in the methods of the invention. In some embodiments, the microorganism is a bacteria, *e*.*g*., *E*. *coli*. Media for growing microorganisms are well known, see, *e*.*g*., Sambrook *et al*. and Current Protocols in Molecular Biology, F.M. Ausubel et al., eds., Current Protocols, a joint venture between Greene Publishing Associates, Inc. and John Wiley & Sons, Inc., (1998 Supplement) (Ausubel). Media can be rich media, *e*.*g*., Luria broth or terrific broth, or synthetic or semi-synthetic medium, *e*.*g*., M9 medium. In some preferred embodiments the growth medium comprises either lactose or globotriose as well as mannose or sialic acid.

"Commercial scale" refers to gram scale production of a sialylated product saccharide in a single reaction. In preferred embodiments, commercial scale refers to production of greater than about 50, 75, 80, 90 or 100, 125, 150, 175, or 200 grams.

The term "operably linked" refers to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence.

A "heterologous polynucleotide" or a "heterologous gene", as used herein, is one that originates from a source foreign to the particular host cell, or, if from the same source, is modified from its original form. Thus, a heterologous sialyltransferase gene in a cell includes a gene that is endogenous to the particular host cell but has been modified. Modification of the heterologous sequence may occur, *e*.*g*., by treating the DNA with a restriction enzyme to generate a DNA fragment that is capable of being operably linked to a promoter. Techniques such as site-directed mutagenesis are also useful for modifying a heterologous sequence.

A "recombinant expression cassette" or simply an "expression cassette" is a nucleic acid construct, generated recombinantly or synthetically, with nucleic acid elements that are capable of affecting expression of a structural gene in hosts compatible with such sequences. Expression cassettes include at least promoters and optionally, transcription termination signals. Typically, the recombinant expression cassette includes a nucleic acid to be transcribed (*e*.*g*., a nucleic acid encoding a desired polypeptide), and a promoter. Additional factors necessary or helpful in effecting expression may also be used. Transcription termination signals, enhancers, and other nucleic acid sequences that influence gene expression, can also be included in an expression cassette. When more than one heterologous protein is expressed in a microorganism, the genes encoding the proteins can be expressed on a single expression cassette or on multiple expression cassettes that are compatible and can be maintained in the same cell. As used herein, expression cassette also encompasses nucleic acid constructs that are inserted into the chromosome of the host microorganism. Those of skill are aware that insertion of a nucleic acid into a chromosome can occur, *e*.*g*., by homologous recombination. An expression cassette can be constructed for production of more than one protein. The proteins can be regulated by a single promoter sequence, as for example, an operon. Or multiple proteins can be encoded by nucleic acids with individual promoters and ribosome binding sites.

Non limitative examples of genes and plasmids used herein are depicted in Table 1 below:
Table 1 : Substrate specificity of lgtD protein. Kinetic parameter for UDP-GalNAc and UDP-Gal in presence of globotriose or globotetraose as acceptor.

**Table 1. Genes, plasmids and Escherichia coli strains used in present invention**

| Plasmids and strains | | |
|---|---|---|
| genes | | |
| *lgtD* | GalNAc transferase from H. influenzae | SEQ ID No 3 |
| | | |
| *gne Cj1131c* | UDP-glucose epimerase from C.jejuni NTCC1168 | CAB73386.1 SEQ ID No 9 |
| | | |
| *futC* | Helicobacter pylori fucosyltransferase | SEQ ID No 5 |
| *gmd, wcaG, manC manB* | *E. coli* genes coding GDP-Man dehydratase, fucose synthase, GDP-Man pyrophosphorylase and Phosphomannomutase respectively | GenBanK U38473 |
| plasmids | | |
| | | |
| pBS-lgtD-gne | pBluescript II SK derivative carrying lgtD and gne | present invention |
| pWKS130 | Cloning vector, Km^{r}, Plac promoter, low copy number, pSC101 replicon | (Wang & Kushner, 1991) |
| pWKS-lgtC | pWKS130 derivative carrying *futC* | present invention |
| pBBRGAB | pBBR1MCS-3 derivative carrying *gmd, wcaG, manC and manB* | (Dumon *et al.,* 2006) |
| | | |
| | | |
| strains | | |
| DC | DH1 *lacZ lacA* | (Dumon *et al.,* 2006) |
| DM | DC *melA* | present invention |
| MR15 | DM (pBS-lgtD-gne) | present invention |
| MR20 | DM (pBS-lgtD-gne, pBBRGAB, pWKS-lgtC) | present invention |
| GLK | DC *galK* | (Dumon *et al.,* 2006) |
| MR16 | GLK (pBS-lgtD-gne) | present invention |
| MR17 | GLK (pBS-lgtD-gne, pBBRGAB, pWKS-lgtC) | present invention |

The term "isolated" refers to material that is substantially or essentially free from components which interfere with the activity biological molecule. For cells, saccharides, nucleic acids, and polypeptides of the invention, the term "isolated" refers to material that is substantially or essentially free from components which normally accompany the material as found in its native state. Typically, isolated saccharides, oligosaccharides, proteins or nucleic acids of the invention are at least about 50%, 55%, 60%, 65%, 70%, 75%, 80% or 85% pure, usually at least about 90%, 91%, 92%, 93%, 94%, 95%, 96%, 97%, 98%, or 99% pure as measured by band intensity on a silver stained gel or other method for determining purity. Purity or homogeneity can be indicated by a number of means well known in the art, such as polyacrylamide gel electrophoresis of a protein or nucleic acid sample, followed by visualization upon staining. For certain purposes high resolution will be needed and HPLC or a similar means for purification utilized. For oligosaccharides, *e*.*g*., sialylated products, purity can be determined using, *e*.*g*., thin layer chromatography, HPLC, NMR or mass spectroscopy.

The terms "identical" or percent "identity," in the context of two or more nucleic acid or polypeptide sequences, refer to two or more sequences or subsequences that are the same or have a specified percentage of amino acid residues or nucleotides that are the same, when compared and aligned for maximum correspondence, as measured using one of the following sequence comparison algorithms or by visual inspection.

As noted above, those of skill will recongize that polynucleotides or polypeptides that are at least substantially identical to those exemplified here can be used in the present invention. The phrase "substantially identical," in the context of two nucleic acids or polypeptides, refers to two or more sequences or subsequences that have at least 60%, preferably 80% or 85%, most preferably at least 90%, 91%, 92%, 93%, 94%, 95%, 96%, 97%, 98%, or 99% nucleotide or amino acid residue identity, when compared and aligned for maximum correspondence, as measured using one of the following sequence comparison algorithms or by visual inspection. Preferably, the substantial identity exists over a region of the sequences that is at least about 50 residues in length, more preferably over a region of at least about 100 residues, and most preferably the sequences are substantially identical over at least about 150 residues. In a most preferred embodiment, the sequences are substantially identical over the entire length of the coding regions.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e*.*g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (*see generally*, Current Protocols in Molecular Biology, F.M. Ausubel et al., eds., Current Protocols, a joint venture between Greene Publishing Associates, Inc. and John Wiley & Sons, Inc., (1995 Supplement) (Ausubel)).

Examples of algorithms that are suitable for determining percent sequence identity and sequence similarity are the BLAST and BLAST 2.0 algorithms, which are described in Altschul et al. (1990) J. Mol. Biol. 215: 403-410 and Altschuel et al. (1977) Nucleic Acids Res. 25: 3389-3402, respectively. Software for performing BLAST analyses is publicly available through the National Center for Biotechnology Information (www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*, *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff, Proc. Natl. Acad. Sci. USA 89:10915 (1989)). In addition to calculating percent sequence identity, the BLAST algorithm also performs a statistical analysis of the similarity between two sequences (*see, e*.*g*., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

"Conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent substitutions" or "silent variations," which are one species of "conservatively modified variations." Every polynucleotide sequence described herein which encodes a polypeptide also describes every possible silent variation, except where otherwise noted. Thus, silent substitutions are an implied feature of every nucleic acid sequence which encodes an amino acid. One of skill will recognize that each codon in a nucleic acid (except AUG, which is ordinarily the only codon for methionine) can be modified to yield a functionally identical molecule by standard techniques. In some embodiments, the nucleotide sequences that encode the enzymes are preferably optimized for expression in a particular host cell (*e*.*g*., yeast, mammalian, plant, fungal, and the like) used to produce the enzymes.

Similarly, "conservative amino acid substitutions," in one or a few amino acids in an amino acid sequence are substituted with different amino acids with highly similar properties are also readily identified as being highly similar to a particular amino acid sequence, or to a particular nucleic acid sequence which encodes an amino acid. Such conservatively substituted variations of any particular sequence are a feature of the present invention. Individual substitutions, deletions or additions which alter, add or delete a single amino acid or a small percentage of amino acids (typically less than 5%, more typically less than 1%) in an encoded sequence are "conservatively modified variations" where the alterations result in the substitution of an amino acid with a chemically similar amino acid. Conservative substitution tables providing functionally similar amino acids are well known in the art. *See, e*.*g*., Creighton (1984) Proteins, W.H. Freeman and Company.

### HOST CELLS

The microorganisms referred herein to practice the invention are recombinant cells. Recombinant cells are generally made by creating or otherwise obtaining a polynucleotide that encodes the particular enzyme(s) of interest, placing the polynucleotide in an expression cassette under the control of a promoter and other appropriate control signals, and introducing the expression cassette into a cell. More than one of the enzymes can be expressed in the same host cells using a variety of methods. For example, a single extrachromosomal vector can include multiple expression cassettes or more that one compatible extrachromosomal vector can be used maintain an expression cassette in a host cell. Expression cassettes can also be inserted into a host cell chromosome, using methods known to those of skill in the art. Those of skill will recognize that combinations of expression cassettes in extrachromosomal vectors and expression cassettes inserted into a host cell chromosome can also be used. Other modification of the host cell, described in detail below, can be performed to enhance production of the desired oligosaccharide. For example, the microorganism may be LacY+ allowing active transport of lactose.

The recombinant cells of the invention are generally microorganisms, such as, for example, yeast cells, bacterial cells, or fungal cells. Examples of suitable cells include, for example, *Azotobacter sp*. (*e*.*g*., *A*. *vinelandii*), *Pseudomonas sp*., *Rhizobium sp*., *Erwinia sp*., *Bacillus sp*., *Streptomyces sp*., *Escherichia sp*. (*e*.*g*., *E*. *coli*), and *Klebsiella sp*., among many others. The cells can be of any of several genera, including Saccharomyces (*e*.*g*., *S*. *cerevisiae*), Candida (*e*.*g*., *C*. *utilis*, *C*. *parapsilosis*, *C*. *krusei*, *C. versatilis, C*. *lipolytica, C*. *zeylanoides, C*. *guilliermondii, C*. *albicans, and C*. *humicola*), Pichia (*e*.*g*., *P*. *farinosa and P*. *ohmeri*), Torulopsis (*e*.*g*., *T*. *candida, T*. *sphaerica, T*. *xylinus, T*. *famata, and T versatilis*), Debaryomyces (*e*.*g*., *D*. *subglobosus, D*. *cantarellii, D*. *globosus, D*. *hansenii, and D*. *japonicus*), Zygosaccharomyces (*e*.*g*., *Z*. *rouxii and Z*. *bailii*), Kluyveromyces (*e*.*g*., *K*. *marxianus*), Hansenula (*e*.*g*., *H*. *anomala and H*. *jadinii*), and Brettanomyces (*e*.*g*., *B*. *lambicus and B*. *anomalus*).

Promoters for use in *E. coli* include the T7, trp, or lambda promoters. A ribosome binding site and preferably a transcription termination signal are also provided. For expression of heterologous proteins in prokaryotic cells other than *E*. *coli,* a promoter that functions in the particular prokaryotic species is required. Such promoters can be obtained from genes that have been cloned from the species, or heterologous promoters can be used. For example, the hybrid trp-lac promoter functions in *Bacillus* in addition to *E. coli.* Methods of transforming prokaryotes other than E. coli are well known. For example, methods of transforming Bacillus species and promoters that can be used to express proteins are taught in U.S. Patent No. 6,255,076 and U.S. Patent No. 6,770,475.

In yeast, convenient promoters include GAL1-10 (Johnson and Davies (1984) Mol. Cell. Biol. 4:1440-1448) ADH2 (Russell et al. (1983) J. Biol. Chem. 258:2674-2682), PHO5 (EMBO J. (1982) 6:675-680), and MFα (Herskowitz and Oshima (1982) in The Molecular Biology of the Yeast Saccharomyces (eds. Strathern, Jones, and Broach) Cold Spring Harbor Lab., Cold Spring Harbor, N.Y., pp. 181-209). Another suitable promoter for use in yeast is the ADH2/GAPDH hybrid promoter as described in Cousens et al., Gene 61:265-275 (1987). For filamentous fungi such as, for example, strains of the fungi Aspergillus (McKnight et al., U.S. Patent No. 4,935,349), examples of useful promoters include those derived from *Aspergillus nidulans* glycolytic genes, such as the ADH3 promoter (McKnight et al., EMBO J. 4: 2093 2099 (1985)) and the *tpiA* promoter. An example of a suitable terminator is the ADH3 terminator (McKnight *et al*.).

In some embodiments, the polynucleotides are placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the glycosyltransferase or enzyme involved in nucleotide sugar synthesis. For *E. coli* and other bacterial host cells, inducible promoters are known to those of skill in the art. These include, for example, the *lac* promoter. A particularly preferred inducible promoter for expression in prokaryotes is a dual promoter that includes a *tac* promoter component linked to a promoter component obtained from a gene or genes that encode enzymes involved in galactose metabolism (*e*.*g*., a promoter from a UDPgalactose 4-epimerase gene (*galE*)).

Inducible promoters for other organisms are also well known to those of skill in the art. These include, for example, the arabinose promoter, the lacZ promoter, the metallothionein promoter, and the heat shock promoter, as well as many others.

The construction of polynucleotide constructs generally requires the use of vectors able to replicate in bacteria. A plethora of kits are commercially available for the purification of plasmids from bacteria. For their proper use, follow the manufacturer's instructions (*see*, for example, EasyPrepJ, FlexiPrepJ, both from Pharmacia Biotech; StrataCleanJ, from Stratagene; and, QIAexpress Expression System, Qiagen). The isolated and purified plasmids can then be further manipulated to produce other plasmids, and used to transfect cells. Cloning in *Streptomyces* or *Bacillus* is also possible.

Selectable markers are often incorporated into the expression vectors used to construct the cells of the invention. These genes can encode a gene product, such as a protein, necessary for the survival or growth of transformed host cells grown in a selective culture medium. Host cells not transformed with the vector containing the selection gene will not survive in the culture medium. Typical selection genes encode proteins that confer resistance to antibiotics or other toxins, such as ampicillin, neomycin, kanamycin, chloramphenicol, or tetracycline. Alternatively, selectable markers may encode proteins that complement auxotrophic deficiencies or supply critical nutrients not available from complex media, *e*.*g*., the gene encoding D-alanine racemase for Bacilli. Often, the vector will have one selectable marker that is functional in, *e*.*g*., *E*. *coli*, or other cells in which the vector is replicated prior to being introduced into the target cell. A number of selectable markers are known to those of skill in the art and are described for instance in Sambrook *et al*., *supra*. A preferred selectable marker for use in bacterial cells is a kanamycin resistance marker (Vieira and Messing, Gene 19: 259 (1982)). Use of kanamycin selection is advantageous over, for example, ampicillin selection because ampicillin is quickly degraded by β-lactamase in culture medium, thus removing selective pressure and allowing the culture to become overgrown with cells that do not contain the vector.

Construction of suitable vectors containing one or more of the above listed components employs standard ligation techniques as described in the references cited above. Isolated plasmids or DNA fragments are cleaved, tailored, and re-ligated in the form desired to generate the plasmids required. To confirm correct sequences in plasmids constructed, the plasmids can be analyzed by standard techniques such as by restriction endonuclease digestion, and/or sequencing according to known methods. Molecular cloning techniques to achieve these ends are known in the art. A wide variety of cloning and *in vitro* amplification methods suitable for the construction of recombinant nucleic acids are well-known to persons of skill.

A variety of common vectors suitable for constructing the recombinant cells of the invention are well known in the art. For cloning in bacteria, common vectors include pBR322 derived vectors such as pBLUESCRIPT™, and λ-phage derived vectors. In yeast, vectors include Yeast Integrating plasmids (*e*.*g*., YIp5) and Yeast Replicating plasmids (the YRp series plasmids) and pGPD-2.

The methods for introducing the expression vectors into a chosen host cell are not particularly critical, and such methods are known to those of skill in the art. For example, the expression vectors can be introduced into prokaryotic cells, including *E*. *coli*, by calcium chloride transformation, and into eukaryotic cells by calcium phosphate treatment or electroporation. Other transformation methods are also suitable.

### METHODS FOR PRODUCING GLOBOSIDES

Production of Sialosyl galactosyl globosides is enhanced by manipulation of the host microorganism. For example, in *E. coli,* break down of sialic acid can be minimized by using a host strain that has diminished CMP-sialic acid synthase acitivity (*NanA*-). In *E. coli,* CMP- sialic acid synthase appears to be a catabolic enzyme. Diminishing the sialic acid degradative pathway in a host cell can be accomplished by disrupting the N-acetylneuraminate lyase gene (NanA, Accession number AE000402 region 70-963). Introduction of a sialyltransferase gene into these mutant strains results in a recombinant cell that is capable of producing large amounts of a sialylated product saccharide.

In some embodiments, the microorganisms are manipulated to enhance transport of an acceptor saccharide into the cell. Here, where lactose or globotriose is the acceptor saccharide, *E. coli* cells that express or overexpress the LacY permease can be used. Also in *E. coli,* when lactose is the acceptor saccharide or an intermediate in synthesizing the sialylated product, lactose breakdown can be minimized by using host cells that are *LacZ*-.

The invention provides methods in which the host cells are used to prepare globosides. As mentioned above, the culture medium may include lactose or globotriose as well as sialic acid or mannose and possibly other precursors to donor substrates or acceptor substrates.

Those of skill will recognize that culture medium for microorganisms can be *e*.*g*., rich mediums, such as Luria broth, animal free Luria broth, or Terrific broth or synthetic medium or semi-synthetic medium, such as M9 medium.

The methods of the invention can be used for producing globosides that are labeled with or enriched in radioisotopes; such oligosaccharides are extremely useful for fundamental biological or conformational analysis studies. The invention thus relates to a method for producing globosides that are labeled with at least one radioisotope. In these embodiments, the culture medium includes substrates labeled said radioisotope and/or in the presence of a said precursor labeled with said radioisotope. The radioisotopes are preferably chosen from the group composed of: 14C, 13C, 3H, 358, 32p, 33p.

The methods of the invention can also be used to activated oligosaccharides that may be used for the chemical synthesis of glycoconjugates or glycopolymers. The lactose acceptor can thus be modified such that glucose residue is replaced with an allyl group, said precursor now being allyl-I3-D galactoside rather than lactose. For example, the double bond of the allyl group is chemically modified by addition, oxidation or ozonolysis reactions.

Methods and culture media for growth of microorganisms are well known to those of skill in the art. Culture can be conducted in, for example, aerated spinner or shaking culture, or, more preferably, in a fermentor.

The products produced by the above processes can be used without purification. However, it is usually preferred to recover the product. Standard, well known techniques for recovery of glycosylated saccharides such as thin or thick layer chromatography, column chromatography, ion exchange chromatography, or membrane filtration can be used. It is preferred to use membrane filtration, more preferably utilizing a reverse osmotic membrane, or one or more column chromatographic techniques for the recovery as is discussed hereinafter and in the literature cited herein.

### THERAPEUTIC AND OTHER USES

Globosides made according to the invention are useful in a wide range of therapeutic and diagnostic applications. They may be used, for example, as an agent for blocking cell surface receptors in the treatment of a host of diseases. As noted above, these oligosaccharides may be used for the chemical synthesis of glycoconjugates (*e*.*g*., glycolipids) or glycopolymers. The oligosaccharides or the glycoconjugates may be used, for example, as nutritional supplements, antibacterial agents, anti-metastatic agents and anti-inflammatory agents. The use of globosides according to the invention as a medicinal product in which the oligosaccharide or glycoconjugate is used to prepare a pharmaceutical composition is thus disclosed herein. Methods for preparing pharmaceutical compositions are well known in the art.

In particular, the present disclosure provides immunoadsorption therapies with the large scale preparation of globosides obtained according the method as defined above. Besides, globopentaose (Gb5) can be used in immunogenic composition for treating various cancers, in particular human embryonal carcinoma cells. Sialosyl galactosyl globoside can be used as an anti-infective agent.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Example 1 : Production of globotriose

A strategy for producing globotriose (**1**) from exogenous lactose is described in **Fig. 1****.** The host strain was an *E. coli* K12 strain JM107 derivative in which the *melA* gene for α-galactosidase activity (melibiase) had been inactivated [21]. The *lgtC* gene, encoding the α-1,4-galactosyltransferase, was amplified from the *Neisseiria meningitidis* L1 (126E) genome and cloned into a pBluescript plasmid. Sequencing revealed a frameshift in *lgtC* due to the deletion of a G in the polyG localised between the 157 bp and 170 bp positions. The reading frame was restored by site directed mutagenesis and the resulting plasmid (pBluescript-*lgtC*) was introduced in JM107*melA⁻* to obtain the globotriose-producing strain TA19. Strain TA19 was cultured to high cell density with glycerol as the carbon and energy source (**Fig.2**).
Lactose (14.6 mM) was added at the beginning of the fed-batch phase at the same time as the inducer IPTG. The production of globotriose (1) was followed by HPAEC-PAD analysis, which showed that lactose rapidly disappeared from the extracellular medium within 10 hours of culture. Lactose consumption correlated with both a small transient intracellular lactose accumulation and the appearance of a longer oligosaccharide, later identified as globotriose (**1**). Interestingly, the intracellular globotriose concentration rapidly plateaued after 3 hours of production whereas the extracellular globotriose concentration steadily increased until all the lactose had been consumed. At this point, a dramatic increase in the intracellular globotriose concentration occurred. Simultaneously a sharp decrease in the extracellular globotriose concentration was observed, suggesting that exogenous globotriose had been taken up by the cells after the complete exhaustion of lactose. Further culture of the cells resulted in a drop in the total globotriose concentration and HPAEC-PAD analysis indicated the simultaneous appearance of a series of longer oligosaccharides.

Cells were harvested after 40 hr of culture and the intracellular fraction was purified by charcoal adsorption and chromatographed on a Biogel P2 column (**Fig.3**)

The separation profile confirmed the presence of a series of at least three compounds (**1, 2** and **3**) which were separated and further characterized. Mass spectral analysis of the purified compounds under the FAB+ mode showed the presence of peaks at m/z 505 and 427 (compound **1**), 667 and 389 (compound **2**), 829 and 851 (compound **3**). These values represented the quasi molecular ions [M+H]⁺ and [M+Na]⁺ of a trihexose (compound **1**), a tetrahexose (compound **2**) and a pentahexose (compound **3**). The identification of compound **1** as globotriose was confirmed by its ¹³C spectrum which showed one C1 of an α-Gal residue at δ=100,68 ppm and was in close agreement with previously reported assignments [10]. The ¹³C spectrum of compound **2** showed two C1 carbons of Gal residues at δ=101.47 ppm and δ= 101.30 ppm, indicating that compound **2** could be identified as Galα-4Galα-4Galβ-4Glc.

### Example 2 : Production of globotetraose from lactose

The system for globotriose production could be extended to the production of globotetraose by additionally expressing the *lgtD* and *wbpP* genes (**Fig.1**). The *lgtD* gene encoding the β 1-3 N-acetylgalactosaminyltransferase activity was amplified from the *Haemophilus influenza* strain Rd DNA and was cloned upstream of *lgtC* in pBluescript-*lgtC* resulting in pBluescript-*lgtDC*. The globotetraose-producing strain TA11 was constructed by co-transforming the JM107*melA⁻* strain with pBluescript-*lgtDC* and pBBR-*wbpP* which is a pBBR1-MCS2 derivative carrying the *Pseudomonas aeruginosa wbpP* gene for UDP-GlcNAc C4 epimerase [22].

The TA11 strain was cultured to a high cell density under the same conditions as for the production of globotriose by the TA19 strain. Globotetraose (**4**) production was monitored by determining the acid hydrolysable hexosamine concentration which increased linearly after the addition of lactose. After 40 hours of culture the final hexosamine concentrations in the intracellular and extracellular fractions were 2.0 mM and 4.5 mM respectively. HPAEC-PAD analysis revealed the presence of several oligosaccharides which could not be properly separated. Similarly, chromatography on Biogel P2 of the intracellular fraction showed a complex mixture of oligosaccharides (**Fig 3**). The major compound (**4**) was further purified by reverse phase HPLC on a HP8NH2/25F column. The mass spectrum of purified compound **4** showed two peaks at m/z 708 and 730, corresponding to the quasi molecular ions [M+H]⁺ and [M+Na]⁺ derived from globotetraose. The ¹³C spectrum was in accordance with previously published data [15] [23] for globotetraose, and the chemical shifts 103.78 ppm, 53.15 ppm, 175.65 ppm and 22.77 ppm assigned to C-1, C-2 and carbons of N-acetyl group respectively clearly, indicated that a GalNAc residue was attached in β anomeric configuration to the globotriose.

To facilitate the identification of the minor compounds, the oligosaccharides of the intracellular fraction were reduced with ABEE. This technique provided an easy UV quantification of oligosaccharide derivatives as well as an amplified MS detection. Six compounds were separated by HPLC. Their molecular ions and characteristic fragments are indicated in Table 2.

**Table 2: Mass spectroscopic characterization of oligosaccharides produced by the TA11 strain after ABEE derivatization and separation by reverse-phase chromatography.**

| ESI positive-ionization data from purified ABEE oligosaccharides (m/z) H⁺ and (Na⁺) | | | | | | |
|---|---|---|---|---|---|---|
| | 5 | 6 | 7 | 8 | 9 | 10 |
| Molecular ions | 654(676) | 816(838) | 978(1000) | 857(879) | 1019(1041) | 1181(1203) |
| Fragment ions | | | | | | |
| Hex-ABEE | 330 | 330 | 330 | 330 | 330 | 330 |
| Hex₂-ABEE | 492(514) | 492(514) | 492 (514) | 492 (514) | 492 (514) | 492 |
| Hex₃-ABEE | | 654(676) | 654 (676) | 654 | 654 (676) | 654 |
| Hex₄-ABEE | | | 816 (838) | | 816 (838) | |
| HexNAc | | | | 204 | 204 | 204 |

Compounds **5, 6** and **7** had molecular weights that corresponded to tri-, tetra and penta-hexose derivatives respectively. This strongly suggested that this series of compounds was identical to the series of galactooligosaccharides which had been previously identified in the culture of the TA19 strain. Compounds **8, 9,** and **10** had the molecular weights of tetra, penta and hexa-hexose derivatives having an hexosamine residue. The formation of the ion fragment m/z 204 from compounds **8, 9** and **10** demonstrates that the hexosamine residue was always located at the non-reducing end of the oligosaccharides. This was confirmed by the formation of the ion fragment m/z 654 (corresponding to the [Hex₃ ABEE]⁺ group) from compound **8** and by the formation of the ion fragment m/z 816 (corresponding to the [Hex₄ ABEE]⁺ group) from compound **9**. Assuming that the hexamine is a β1-3 linked GalNAc added onto globotriose and its mono and di-galactosylated derivatives, the three GalNAc containing oligosaccharides produced by the TA11 strain can thus be identified as
GalNAcβ-3Galα-4Galβ-4Glc (**8**),
GalNAcβ-3Galα-4Galα-4Galβ-4Glc (**9**) and
GalNAcβ-3Galα-4Galα-4Galα-4Galβ-4Glc (**10**)

### Example 3: Production of globotetraose from globotriose

The observation that extracellular globotriose was taken up by the cells after the complete exhaustion of lactose during the culture of the TA19 strain led us to investigate the possibility of using globotriose (**1**) as an exogenous acceptor for globotetraose synthesis as illustrated in **Fig. 4**.

The globotetraose-producing strain TA21 was constructed by co-transforming the JM107*melA* strain with pBBR-*wbpP* and pACT3-*lgtD*. The TA21 strain was cultured at high cell density and globotriose (1) (1.27mM) was added instead of lactose as the acceptor. Analysis of oligosaccharide content in the intracellular and the extracellular fraction by thin layer chromatography indicated that globotriose (**1**) was rapidly internalized and entirely converted into globotetraose (**4**) within 6 hours of culture (**Fig. 5**).

Its final yield was estimated to be 1.25 mM by colorimetric quantification of acid-hydrolysable hexosamine. Since it was the only oligosaccharide remaining at the end of the culture, the globotetraose (**4**) was easily purified from the intracellular fraction by chromatography on Biogel P2 and its identity was confirmed by mass spectrometry.

### Example 4: Production of globotriose without polygalactosylated side-products

The polygalactosylated tetrasaccharide (Galα-4Galα-4Galβ-4Gal) and pentasaccharide (Galα-4Galα-4Galα-4Galβ-4Gal) were found in large amount in the cells of strain TA19 that were harvested after 40 hours of culture (**Fig**.**3**). The formation of these side-products has two detrimental impacts on the process of globotriose production by the strain TA19. First it reduces the overall globotriose yield. Secondly it makes the globotriose purification more difficult to achieve.

To improve the globotriose yield we have increased the concentration of lactose to 7.5 g.L-1 and carefully monitored the formation of oligosaccharide by TLC analysis. The **Fig.6** shows that globotriose was the only oligosaccharide produced by strain TA19 during the first 8 hours that followed the addition of lactose. After 8 hours, lactose was almost entirely consumed and the culture was stopped to prevent a further galactosylation of globotriose.

Globotriose was recovered from both the intracellular and the extracelluar fraction of the 8 hours cultures of strain TA19 and purified by adsorption on activated charcoal. From a 7 liter culture of strain TA19, 35 g of charcoal purified globotriose fraction were obtained. Chromatography on Biogel P2 showed the presence of small amount of residual lactose but confirmed the absence of contaminating polygalactosylated compounds and the presence of globotriose as the major product (**Fig 7****.**)

### Example 5: Production of globopentaose (Stage Specific Embryonic Antigen-3 SSEA-3)

As described above in example 3, globotriose is rapidly converted into globotetraose by the strain TA21 and, after 6 hours of incubation, globotetraose was the major sugar recovered by chromatography on Biogel P2 from the intracellular oligosaccharide fraction of strain TA21 culture. However, the Biogel P2 chromatogram showed the presence of a small amount of an unidentified sugar (peak 3 **Fig. 8A**) which was larger than the globotetraose.

The strain TA21 was cultivated for a prolonged period of time and the analysis by chromatography on Biogel P2 showed that, after 20 hours of culture, the unidentified sugar has become the major compound detected in the intracellular fraction (Fig 8B). Concurrently a decrease in the globotetraose concentration was also observed. The ESI⁺ mass spectrum of the peak 3 fraction showed the presence of a quasi molecular ions [M+Na]⁺ at m/z 892 which could originated from a pentasaccharide having 4 hexose residues and one *N*-acetylhexosamine residue. The determination of the monomeric sugar composition by HPAEC-PAD analysis after acid hydrolysis indicated that the pentasaccharide was made of Glc, Gal and GalNAc in a ratio of 1/3/1. This strongly suggests that the pentasaccharide was formed by the transfert of one Gal residue on a molecule of globotetraose. Compared with the NMR spectrum of the globotetraose, the ¹³C NMR spectrum of the pentasaccharide showed an additional signal at 105.6 ppm indicating that the third Gal residue was attached on the GalNAc with a β linkage. The linkage position on the GalNAc was determined by GC-MS analysis after methylation, acid-hydrolysis, reduction and acetylation of the pentasaccharide. The presence of fragment ions at m/z 261 and 161 derived from the GalNAc residue unambiguously demonstrates that the GalNAc was substituted on the 3 position.

These results clearly show that the pentasaccharide produced by strain TA21 after 20 hours of culture has the structure of the globopentaose: Galβ-3GalNAcβ-3Galα-4Galβ-4Gal. Since *E. coli* K12 is not known to have a β-3 Gal transferase activity, it is very likely that the conversion of globotriose into globotetraose has been catalysed by LgtD which is the only heterologous glycosyltransferase overexpressed in strain TA21. LgtD is thus a bifunctional glycosyltransferase having a β-3 GalNAc activity when globotriose is the acceptor and a β-3 Gal transferase activity when globotetraose is the acceptor. In addition, enzymatic assays with crude extracts from a strain overexpressing the *lgtD* gene indicated that the LgtD protein had both a galactosyl- and a *N*-acetylgalactosaminyl-transferase activity in presence of globotriose or globotetraose as acceptor. The maximum.velocity rates of these two activities were in the same range whatever the acceptor and the sugar donor. However, large differences in the affinity of the enzyme for UDP-Gal or UDP-GalNAc were observed as a function of the acceptor used. When globotriose was the acceptor, the Km was 6 fold lower for UDP-GalNAc than for UDP-Gal indicating that lgtD act primarily as a GalNAc transferase converting globotriose into globotetraose. Replacement of globotriose by globotetraose as the acceptor resulted in 20 fold increase in the Km for UDP-GalNAc but on the contrary in a 3.5 fold decrease in the Km for UDP-Gal. In presence of globotetraose as the acceptor the enzyme, which had an 11 fold better affinity for UDP-Gal than for UDP-GalNAc, can thus be regarded as a galactosyltransferase which specifically direct the synthesis of globopentaose.

It has already been reported that some GalNAc transferases have a low Gal transferase activity but they normaly have the same specificity for the acceptor whatever UDP-Gal or UDP GalNAc was used as sugar donor. On the contrary the acceptor specificities of the two glycosyltransferase activities of LgtD are very high because we never detected the formation of a tetrasaccharide different from globotetraose and of a pentasaccharide different from globopentaose. To our knowledge, the specific β-3gal transferase activity of LgtD with globotetraose as the acceptor has never been reported before.

The *lgtD* gene (HI1578) from *Haemophilus influenzae* strain rd can thus be advantageously used for the conversion of globotriose into globopentaose by using a metabolically engineered strain that is devoid of α and β galactosidase activity, and coexpresses the *lgtD* gene with a gene encoding a UDP-GlcNAc C4 epimerase activity as illustrated in **Fig 9**.

### Example 5 bis: optimization of globotetraose and globopentaose production

In order to optimize the globopentaose production, the *wbpP* gene was replaced by the *C*. *jejuni gne* gene, which has been shown to encode a more active UDP-GlcNAc C4 epimerase (Bernatchez *et al*., 2005), The *gne* gene was cloned by PCR from the genomic DNA of *C*. *jejuni* strain NCTC 11168 and both *gne* and *lgtD* were cloned together on the high copy number pBluescript plasmid to yield pBS-lgtD-gne.

TLC analysis of the intracellular oligosaccharide content of strain MR15 indicated that the globotetraose production rate was significantly improved when compared with strain TA21 with carried the plasmids pACT3-lgtD and pBBR-wbpP. Globotriose (3 g.1⁻¹) was entirely converted into glotetraose within 4 hours of incubation by strain MR15, whereas it took 6 hours for the strain TA21 to convert only 1 g.l⁻¹ of globotriose. After purification by charcoal adsorption and size exclusion chromatography the yield of pure globopentaose was 1.29 g from a one liter culture.

### Example 6: Production of Globo-H hexasaccharide

We have already shown that fucosylated oligosaccharide can be produced in living *E. coli* cells that have been metabolically engineered to overexpress the genes involved in GDP-Fucose biosynthesis and the appropriate glycosyltransferase genes [26]. It has also been reported that the *Helicobacter pylori* gene *futC* which encodes an α-2 fucosyltransferase is functionnaly expressed in *E. coli* [25]. Therefore, we provide a new method for producing the Globo-H hexasaccharide (Fucα-2Galβ-3GalNAcβ-3Galα-4Galβ-4Gal) from globotriose using a strain that overexpresses the *lgtD* and *futC* genes as illustrated in **Fig. 10**. In one embodiment, to prevent a possible fucosylation of the globotriose, a mutant *manA⁻* devoid of phophomannose isomerase activity can be used. This mutant will be unable to synthesize GDP-Fuc, unless mannose is exogenously added in the medium and taken up and phosphorylated in Man-6-P by the mannose permease encoded by the *manXXZ* genes. By adding the mannose after the entire conversion of globotriose into globopentaose, the fucosylation of globotriose will be impossible and all the globotriose will be converted into Globo-H oligosaccharide. In another preferred embodiment, the strain MR20 was constructed by transforming the host strain DM with the three plasmids pBS-lgtD-gne, pBBRGAB and pWKS-lgtC. The host strain DM was a *melA* null derivative of strain DC (Dumon *et al*., 2006). The plasmid pBBRGAB contained the four genes *gmd*, *wcaG*, *manC manB* required for the synthesis of GDP-Fuc (Dumon *et al*., 2006). The plasmid pWKS-lgtC was constructed by cloning the *futC* gene from pEXT20futC (Drouillard *et al.,* 2006) into the *KpnI SalI* sites of pWKS130 yielding pWKS-futC. The strain MR20, was cultivated in presence of 3 g.l⁻¹ of globotriose. TLC analysis shows that, after 23 h of incubation, globotriose was entirely converted into globotetraose (**3**) and a compound (**5**) that migrates as an hexaose (**Fig. 11**). Prolonged incubation resulted in the almost complete conversion of Globotetraose into compound (**5**). Compound (**5**) was purified on activated charcoal and by size exclusion chromatography on Biogel P2 with a yield of 1.59 gram from a one liter culure and its identification as globo-H sugar was confirmed by mass spectrometry and NMR analysis. During the culture there was no accumulation of Globopentaose indicating that the fucosyltransferase was very active on globopentaose. On the other hand there was no significant formation of a compound that could migrate as a fucosylated globotriose suggesting that Globotriose was not a good substrate for the fucosyltransferase.

### Example 7: Production of sialosyl galactosyl globoside (SGG) hexasaccharide (Stage Specific Embryonic Antigen-4, SSEA-4)

It has already been reported that 3'sialyllactose (NeuAcα-3Gαlβ-4Glc) can be produced from exogenous lactose and sialic acid (NeuAc) by metabolically engineered living *E. coli* cells that overexpressed heterologous gene *nst* for α-3 sialyltransferase and for the CMP-NeuAc synthase (17). We have combined this sialylation system with the system of globopentaose synthesis as described in example 5 to produce the SGG hexasaccharide (NeuAcα-3Galβ-3GalNAcβ-3Galα-4Galβ-4Gal) from exogenous globotriose and NeuAc as shown in Fig 12. To prevent a possible sialylation of the globotriose, NeuAc is added after the entire conversion of globotriose into globotetraose.

### Example 8: Production of the terminal epitope of the SSEA-3, globo-H antigen and SSEA-4 using Gal as acceptor.

A *galk* mutant lacking galactokinase activity can use galactose as acceptor for the synthesis of oligosaccharide with a terminal gal residue. The strain MR16 was constructed by transforming the lacZ⁻ galK⁻ host strain host GLK with the pBS-lgtD-gne plasmid. Culture of strain MR16 in presence of galactose resulted in the formation of the terminal trisaccharide structure of the SSEA-3 antigen (Galβ-3GalNAcβ-3Gal) with a transient accumulation of the disaccharide intermediate GalNAcβ-3Gal. These results demonstrate that the LgtD act as GalNAc transferase in presence of Gal and as a Gal transferase in presence of GalNAcβ-3Gal.
This method can be extended to the production of the terminal tetrasaccharide structure of the Globo-H antigen (Fucα-2Galβ-3GalNAcβ-3Gal) as described in Figure 13. Similarly, the system for the synthesis of the sialosyl galactosyl globoside (SGG) hexasaccharide described in example 7 can be adapted to the synthesis of the terminal tetrasaccharide epitope of the SSEA-4 antigen (NeuAcα-3Galβ-3GalNAcβ-3Gal) by using Gal as the acceptor.

### Example 9: Production of Globo-H hexasaccharide with a terminal propargyl proup

The strain MR20 was cultivated as in example 6 except that propargyl-β-globotrioside (1 g.l⁻¹) was used as acceptor instead of globotriose. At the end of the culture the propargyl-β-globotrioside was entirely consumed and the main oligosaccharide product was a compound that migrated in TLC as an hexasaccharide and which was purified by charcoal adsorption and size exclusion chromatography on Biogel P2 with a yield of 0.53 gram from a one liter culture. This compound was identified as Fucα-2Galβ-3GalNAcβ-3Galβ-4Galβ-4Galβ-propargyl by mass spectrometry (EI-MS: *m*/*z* [M+Na]⁺ = 1076,) and NMR analysis. Selected ¹H signals
¹H-NMR (D₂O, 293K): δ (ppm) = 5.24 (H-1""', *d, J* = 2Hz, 1H), 4.90 (H-1", *d, J* = 2Hz, 1H), 4.68, 4.63, 4.55, 4.52 (H-1, H-1', H-1"', H-1"", 4d, *J* = 7.8Hz, 4H), 4.51 (CH₂ Prop, s, 2H), 3.35 (H-2β, m, *J* = 7.8Hz, 1H), 2.04 (COCH₃, s, 3H), 1.23 (H-6""', d, *J* = 6.8Hz, 3H). ¹³C NMR (D₂O, 303K): δ (ppm) = 175.48 (CO), 105.16, 104.49, 103.25, 101.64, 101.57 (C-1, C-1', C-1", C-1"', C-1"",), 100.45 (C-1""'), 57.78 (CH₂Prop), 52.84 (C-2"'), 23.46 (CH₃), 16.50 (C-6""').

### Example 10: Production of Globo-H tetrasaccharide with a terminal propargylproup

The strain MR17 was cultivated as in example 8 except that propargyl-β-galactose (3 g.l⁻¹) was used as acceptor instead of galactose. The major oligosaccharide recovered at the end of the culture migrated as a tetrasaccharide in TLC and was purified with a yield of 1.65 g from a one liter culture. This compound was identified as Fucα-2Galβ-3GalNAcβ-3Gal1β-propargyl by mass spectrometry (EI-MS: *m*/*z* [M+Na]⁺ = 752) and NMR analysis. Selected ¹H signals¹H-NMR (D₂O, 293K): δ (ppm) = 5.24 (H-1"', d, *J* = 2Hz, 1H), 4.62, 4.56 (H-1, H-1', H-1", 2d, *J* = 7.8Hz, 3H), 4.48 (CH₂ Prop, s, 2H), 4.25 (H-5"', q, *J* = 6.8Hz, 1H), 2.04 (COCH₃, s, 3H), 1.23 (H-6"', d, *J* = 6.8Hz, 3H). ¹³C NMR (D₂O, 303K): δ (ppm) = 175.52 (CO), 105.25, 103.21, 102.58 (C-1, C-1', C-1"), 100.50 (C-1"'), 57.82 (CH₂Prop), 52.89 (C-2'), 23.54 (CH₃), 16.50 (C-6"').

### REFERENCES

[1] A. A. Lindberg, J. E. Brown, N. Stromberg, M. Westling-Ryd, J. E. Schultz, and K. A. Karlsson, Identification of the carbohydrate receptor for Shiga toxin produced by Shigella dysenteriae type 1, J Biol Chem 262 (1987) 1779-1785.
[2] K. E. Brown, S. M. Anderson, and N. S. Young, Erythrocyte P antigen: cellular receptor for B19 parvovirus, Science 262 (1993) 114-117.
[3] P. R. Chipman, M. Agbandje-McKenna, S. Kajigaya, K. E. Brown, N. S. Young, T. S. Baker, and M. G. Rossmann, Cryo-electron microscopy studies of empty capsids of human parvovirus B19 complexed with its cellular receptor, Proc Natl Acad Sci USA 93 (1996) 7502-7506.
[4] J. A. Roberts, B. I. Marklund, D. liver, D. Haslam, M. B. Kaack, G. Baskin, M. Louis, R. Mollby, J. Winberg, and S. Normark, The Gal(alpha 1-4)Gal-specific tip adhesin of Escherichia coli P-fimbriae is needed for pyelonephritis to occur in the normal urinary tract, Proc Natl Acad Sci U S A 91 (1994) 11889-11893.
[5] R. J. Scholten, B. Kuipers, H. A. Valkenburg, J. Dankert, W. D. Zollinger, and J. T. Poolman, Lipo-oligosaccharide immunotyping of Neisseria meningitidis by a whole-cell ELISA with monoclonal antibodies, J Med Microbiol 41 (1994) 236-243.
[6] E. C. Gotschlich, Genetic locus for the biosynthesis of the variable portion of Neisseria gonorrhoeae lipooligosaccharide, J Exp Med 180 (1994) 2181-2190.
[7] M. P. Jennings, D. W. Hood, I. R. Peak, M. Virji, and E. R. Moxon, Molecular analysis of a locus for the biosynthesis and phase-variable expression of the lacto-N-neotetraose terminal lipopolysaccharide structure in Neisseria meningitidis, Mol Microbiol 18 (1995) 729-740.
[8] W. W. Wakarchuk, A. Cunningham, D. C. Watson, and N. M. Young, Role of paired basic residues in the expression of active recombinant galactosyltransferases from the bacterial pathogen Neisseria meningitidis, Protein Eng 11 (1998) 295-302.
[9] K. F. Johnson, Synthesis of oligosaccharides by bacterial enzymes, Glycoconj J 16 (1999) 141-146.
[10] J. Zhang, P. Kowal, J. Fang, P. Andreana, and P. G. Wang, Efficient chemoenzymatic synthesis of globotriose and its derivatives with a recombinant alpha-(1-->4)-galactosyltransferase, Carbohydr Res 337 (2002) 969-976.
[11] S. Koizumi, T. Endo, K. Tabata, and A. Ozaki, Large-scale production of UDP-galactose and globotriose by coupling metabolically engineered bacteria, Nat Biotechnol 16 (1998) 847-850.
[12] J. Zhang, P. Kowal, X. Chen, and P. G. Wang, Large-scale synthesis of globotriose derivatives through recombinant E. coli, Org Biomol Chem 1 (2003) 3048-3053.
[13] A. Risberg, G. Alvelius, and E. K. Schweda, Structural analysis of the lipopolysaccharide oligosaccharide epitopes expressed by Haemophilus influenzae strain RM.118-26, Eur J Biochem 265 (1999) 1067-1074.
[14] D. W. Hood, A. D. Cox, W. W. Wakarchuk, M. Schur, E. K. Schweda, S. L. Walsh, M. E. Deadman, A. Martin, E. R. Moxon, and J. C. Richards, Genetic basis for expression of the major globotetraose-containing lipopolysaccharide from H. influenzae strain Rd (RM118), Glycobiology 11 (2001) 957-967.
[15] J. Shao, J. Zhang, P. Kowal, and P. G. Wang, Donor substrate regeneration for efficient synthesis of globotetraose and isoglobotetraose, Appl Environ Microbiol 68 (2002) 5634-5640.
[16] J. Shao, J. Zhang, P. Kowal, Y. Lu, and P. G. Wang, Efficient synthesis of globoside and isogloboside tetrasaccharides by using beta(1-->3) N-acetylgalactosaminyltransferase/UDP-N-acetylglucosamine C4 epimerase fusion protein, Chem Commun (Camb) (2003) 1422-1423.
[17] B. Priem, M. Gilbert, W. W. Wakarchuk, A. Heyraud, and E. Samain, A new fermentation process allows large-scale production of human milk oligosaccharides by metabolically engineered bacteria, Glycobiology 12 (2002) 235-240.
[18] D. M. Dykxhoorn, R. St Pierre, and T. Linn, A set of compatible tac promoter expression vectors, Gene 177 (1996) 133-136.
[19] D. T. Li, and G. R. Her, Structural analysis of chromophore-labeled disaccharides and oligosaccharides by electrospray ionization mass spectrometry and high-performance liquid chromatography/electrospray ionization mass spectrometry, J Mass Spectrom 33 (1998) 644-652.
[20] S. Suzuki, K. Kakehi, and S. Honda, Comparison of the sensitivities of various derivatives of oligosaccharides in LC/MS with fast atom bombardment and electrospray ionization interfaces, Anal Chem 68 (1996) 2073-2083.
[21] E. Bettler, A. Imberty, B. Priem, V. Chazalet, A. Heyraud, D. H. Joziasse, and R. A. Geremia, Production of recombinant xenotransplantation antigen in Escherichia coli, Biochem Biophys Res Commun 302 (2003) 620-624.
[22] T. Antoine, B. Priem, A. Heyraud, L. Greffe, M. Gilbert, W. W. Wakarchuk, J. S. Lam, and E. Samain, Large-scale in vivo synthesis of the carbohydrate moieties of gangliosides GM1 and GM2 by metabolically engineered Escherichia coli, Chembiochem 4 (2003) 406-412.
[23] U. Nilsson, A. K. Ray, and G. Magnusson, Synthesis of the globotetraose tetrasaccharide and terminal tri- and di-saccharide fragments, Carbohydr Res 252 (1994) 117-136.
[24] G. Cornelis, R. K. Luke, and M. H. Richmond, Fermentation of raffinose by lactose-fermenting strains of Yersinia enterocolitica and by sucrose-fermenting strains of Escherichia coli, J Clin Microbiol 7 (1978) 180-183.
[25] Wang, G., P. G. Boulton, et al. (1999). "Novel Helicobacter pylori alpha1,2-fucosyltransferase, a key enzyme in the synthesis of Lewis antigens." Microbiology 145 (Pt 11): 3245-53.
[26] Dumon, C., B. Priem, et al. (2001). "In vivo fucosylation of lacto-N-neotetraose and lacto-N-neohexaose by heterologous expression of Helicobacter pylori alpha-1,3 fucosyltransferase in engineered Escherichia coli." Glycoconj J 18(6): 465-74.
[27] Wenk, J., P. W. Andrews, et al. (1994). "Glycolipids of germ cell tumors: extended globo-series glycolipids are a hallmark of human embryonal carcinoma cells." Int J Cancer 58(1): 108-15.
[28] Slovin, S. F., G. Ragupathi, et al. (1999). "Carbohydrate vaccines in cancer: immunogenicity of a fully synthetic globo H hexasaccharide conjugate in man." Proc Natl Acad Sci U S A 96(10): 5710-5.
[29] Krupnick, J. G., I. Damjanov, et al. (1994). "Globo-series carbohydrate antigens are expressed in different forms on human and murine teratocarcinoma-derived cells." Int J Cancer 59(5): 692-8.
[30] Stapleton, A. E., M. R. Stroud, et al. (1998). "The globoseries glycosphingolipid sialosyl galactosyl globoside is found in urinary tract tissues and is a preferred binding receptor In vitro for uropathogenic Escherichia coli expressing pap-encoded adhesins." Infect Immun 66(8): 3856-61
[31] Ito, A., S. Saito, et al. (2001). "Monoclonal antibody (5F3) defining renal cell carcinoma-associated antigen disialosyl globopentaosylceramide (V3NeuAcIV6NeuAcGb5), and distribution pattern of the antigen in tumor and normal tissues." Glycoconj J 18(6): 475-85.

## Claims

1. The use of the glycosyltransferase encoded by the *lgtD* gene from Haemophilus influenzae of SEQ ID No 3 or a sequence having at least 80% identity thereof, as a β1,3 galactosyl transferase to catalyze the transfer of a galactose moiety from UDP-Gal to an acceptor bearing the terminal non reducing structure GalNAcβ-3-R to form the Galβ-3GalNAcβ-3-R structure, wherein R is selected from the group consisting of galactose, galactose-X, β galactosides such as allyl-β-galactoside or propargyl-β-galactoside, α galactosides, globotriose, β globotrioside such as allyl-β-globotrioside or propargyl-β-globotrioside, α globotrioside, X being defined as a reactive group allowing the covalent coupling with another molecule, including amino, azide and nitrophenyl groups.

2. The use according to claim 1, to produce an oligosaccharide selected from Galβ-3GalNAcβ-3Gal, Galβ-3GalNAcβ-3Galα-X, Galβ-3GalNAcβ-3Galβ-X, Galβ-3GalNAcβ-3Galβ-allyl, Galβ-3GalNAcβ-3Galβ-propargyl, Galβ-3GalNAcβ-3Galα-4Galβ-4Gal (Globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Gaβ-4Galβ-allyl, and Galβ-3GalNAcβ-3Galα-4Galβ-4Galβ-propargyl.

3. The use of a microorganism comprising a heterologous *lgtD* gene from Haemophilus influenzae of SEQ ID No 3 or a sequence having at least 80% identity thereof to produce an oligosaccharide selected from Galβ-3GalNAcβ-3Gal, Galβ-3GalNAcβ-3Gala-X, Galβ-3GalNAcβ-3Galβ-X, Galβ-3GalNAcβ-3Galβ-allyl, Galβ-3GalNAcβ-3Galβ-propargyl, Galβ-3GalNAcβ-3Galα-4Galβ-4Gal (Globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Galβ-4Galβ-allyl, and Galβ-3GalNAcβ-3Galα-4Galβ-4Galβ-propargyl.

4. The use according to claim 3 of a microorganism which is *LacY*+, *LacZ*-, *melA*- and comprises a heterologous *LgtD* gene from Haemophilus influenzae of SEQ ID No 3 or a sequence having at least 80% identity thereof and encoding β-3 GalNAc transferase, and a *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C. jejuni* strain NCTC 11168 of SEQ ID No 9 for producing Galβ-3GalNAcβ-3Galα-4Galβ-4Gal (Globopentaose).

5. A method for producing Galβ-3GalNAcβ-3Galα-4Galβ-4Gal (Globopentaose), comprising the step of culturing a microorganism in a culture medium comprising globotriose (Galα-4Galβ-4Glc), wherein said microorganism comprises a heterologous *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C. jejuni* strain NCTC 11168 of SEQ ID No 9 and a heterologous *lgtD* gene from Haemophilus influenzae of SEQ ID No 3 or a sequence having at least 80% identity thereof and encoding β-3 GalNAc transferase.

6. A method for producing Galβ-3GalNAcβ-3Galα-4Galβ-4Gal (Globopentaose), comprising the step of culturing a microorganism in a culture medium comprising lactose, wherein said microorganism comprises a heterologous *lgtC* gene encoding α-1,4-Gal transferase which transfers a galactose moiety from UDP-Gal to the lactose to form globotriose (Galα-4Galβ-4Glc), a heterologous *lgtD* gene from Haemophilus influenzae of SEQ ID No 3 or a sequence having at least 80% identity thereof and encoding β-3 GalNAc transferase, and a heterologous *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of C. *jejuni* strain NCTC 11168 of SEQ ID No 9.

7. The method according to claim 5 or 6, wherein the *lgtD* gene transfers a GalNAc moiety from UDP-GalNAc to globotriose to form globotetraose (GalNAcβ-3-Galα-4Galβ-4Glc) as well as a galactose moiety from UDP-Gal to globotetraose to form globopentaose (Galβ-3GalNAcβ-3Galα-4Galβ-4Gal).

8. The method according to claim 5, wherein globotriose is obtained from a method comprising culturing a microorganism which is *LacY+* (β-galactoside permease), *LacZ-*(β galactosidase), and *MelA*- (β-galactosidase) in a culture medium comprising lactose, wherein said microorganism comprises a heterologous *lgtC* gene encoding α-1,4-Gal transferase which transfers a galactose moiety from UDP-Gal to the lactose to form globotriose (Galα-4Galβ-4Glc) and wherein lactose is in excess in the culture medium.

9. The method according to claim 8, wherein the culture is terminated before the exhaustion of lactose and globotriose is extracted from the culture medium.

10. The method according to claim 8, wherein the *LgtC* gene is from *Neisseria meningititis.*

11. The method according to one of claims 5 to 8, wherein said microorganism is *LacY+* (β-galactoside permease), *LacZ-* (β galactosidase), *melA*- (α-galactosidase).

12. The method according to one of claims 5 to 8, wherein said microorganisms are recombinant *E. coli* strains.

13. The method according to one of claims 5 to 8, wherein glycerol is used in the media of said microorganisms as carbon and energy source.

14. A set of two separate microorganisms, comprising said microorganism as defined in claim 5 and a microorganism as defined in claim 8.

15. A cell culture comprising globotriose and a microorganism of claim 5.

16. The use of a microorganism of claim 5 or 6 to produce high yield globopentaose in cell culture.

17. The use according to claim 1 of the *lgtD* gene from *Haemophilus influenzae* of SEQ ID No 3 or of a sequence displaying at least 80% identity thereof and encoding β-3 GalNAc transferase, to catalyze the transfer of a galactose moiety from UDP-Gal to globotetraose to form globopentaose (β-3 Gal transferase activity).

18. The method according to claim 5 or 6, wherein said microorganism further comprises a heterologous *futC* gene encoding an α-2 fucosyltransferase to transfer a fucose moiety from GDP-Fuc to globopentaose to form Globo-H hexasaccharide (Fucα-2Galβ-3GalNAcβ-3Galα-4Galβ-4Gal).

19. A microorganism as defined in claim 18, which is *LacY*+, (optionally *Mel*A-, *manXXZ*+*)*, *manA⁻* and which comprises a heterologous *lgtD* gene from *Haemophilus influenzae* of SEQ ID No 3 or a sequence having at least 80% identity thereof (β-3 GalNAc transferase), a heterologous a *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C. jejuni* strain NCTC 11168 of SEQ ID No 9 and a heterologous *futC* gene (α-2 fucosyltransferase), such as the *Helicobacter pylori* gene *futC* of SEQ ID No 5.

20. A set of two separate microorganisms, comprising said microorganism as defined in claim 19 and a microorganism comprising a heterologous *lgtC* gene encoding α-1,4-Gal transferase and which is *LacY*+ (β-galactoside permease), *LacZ*- (β galactosidase), and MeM- (α-galactosidase).

21. The method according to claim 5 or 6, wherein said microorganism further comprises a gene encoding the CMP-NeuAc synthase, such as a gene encoding the CMP-NeuAc synthase from *N*. *meningitidis*, and a heterologous gene encoding α-3 sialyltransferase, such as the α-3 sialyltransferase gene from *N*. *meningitidis* of SEQ ID No 7, which catalyzes the transfer of a sialyl moiety from an activated sialic acid molecule to globopentaose to form sialosyl galactosyl globoside (SGG) hexasaccharide (NeuAcα-3Galβ-3GalNAcβ-3Galα-4Galβ-4Gal).

22. The method according to claim 21, wherein NeuAc is added in the medium after the entire conversion of globotriose into globopentaose.

23. The microorganism as defined in claim 21 which is *LacY*+, *MelA*-, *nanT*+, *nanA⁻* and which comprises a heterologous *LgtD* gene from *Haemophilus influenzae* of SEQ ID No 3 or a sequence having at least 80% identity thereof (β-3 GalNAc transferase), a heterologous a *wbpP* encoding for UDP-GlcNAc-C4 epimerase, such as the *Pseudomonas aeruginosa wbpP* gene or a *gne* gene encoding for a UDP-glucose 4-epimerase, such as the *gne* gene of *C. jejuni* strain NCTC 11168 of SEQ ID No 9 and a heterologous gene for α-3 sialyltransferase, such as the gene from N. meningitidis of SEQ ID No 7.

24. A cell culture medium comprising the microorganism as defined in claim 23 and sialic acid.

25. A set of two separate microorganisms, comprising said microorganism as defined in claim 23 and a microorganism comprising a heterologous *lgtC* gene encoding α-1,4-Gal transferase and which is *LacY*+ (β-galactoside permease), *LacZ*- (β galactosidase), and *MelA*- (α-galactosidase).

26. A method for producing an oligosaccharide comprising the galabiose motif (Galα-4Gal), referred as globosides, selected from the group consisting of globopentaose, and galactosyl-globosides including globo-H hexasaccharide, sialosyl galactosyl globoside (SGG) hexasaccharide, comprising the step consisting of culturing a microorganism as defined in claim 5, 6, 19, or 23.

27. A cell culture comprising globotriose at a concentration of 1 to 10 g.L¹ and a microorganism as defined in claim 5, 19, or 23.

28. The use according to claim 1 of a heterologous *lgtD* gene from *Haemophilus influenza* of SEQ ID No 3 or a sequence having at least 80% identity thereof, as a Gal transferase in presence of GalNAcβ-3Gal to form the SSEA-3 antigen (Galβ-3GalNAcβ-3Gal).

29. A method for producing an oligosaccharide comprising the motif GalNAcβ-3Gal, comprising culturing a microorganism which is *galP* (galactose permease), *LacZ-* (β galactosidase), *MelA*- (α-galactosidase) and *wbpP* encoding for UDP-GlcNAc-C4 epimerase, such as the *Pseudomonas aeruginosa wbpP* gene ; or a *gne* gene encoding for a UDP-glucose 4-epimerase, such as the *gne* gene of *C. jejuni* strain NCTC 11168 of SEQ ID No 9, in a culture medium comprising galactose, wherein:
• said microorganism comprises a heterologous *IgtD* gene from *Haemophilus influenzae* of SEQ ID No 3 or a sequence having at least 80% identity thereof and encoding α-1,4-Gal transferase which transfers a GalNAc residue to galactose to form an oligosaccharide comprising GalNAcβ-3Gal, and
• the *lgtD* gene product allowed to further transfer a galactose moiety from UDP-Gal to GalNAcβ-3Gal to form the SSEA-3 antigen (Galβ-3GalNAcβ-3Gal).

30. The method according to claim 29, which is extended to the production of the terminal tetrasaccharide epitope of the Globo-H antigen (Fucα-2Galβ-3GalNAcβ-3Gal) and wherein the microorganism further comprises a heterologous *futC* gene encoding an α-2 fucosyltransferase to transfer a fucose moiety from GDP-Fuc to Galβ-3GalNAcβ-3Gal to form Fucα-2Galβ-3GalNAcβ-3Gal.

31. A microorganism as defined in claim 29.

32. A microorganism as defined in claim 30.

33. A culture medium comprising galactose and a microorganism of claim 31 or 32.

## Patentansprüche

1. Verwendung der Glycosyltransferase, codiert durch das *lgtD-Gen* von Haemophilus influenzae von SEQ ID Nr. 3 oder einer Sequenz mit mindestens 80 % Identität damit, als eine β1,3 Galaktosyltransferase, um die Übertragung eines Galaktoseanteils von UDP-Gal an einen Akzeptor zu katalysieren, der die terminale nicht reduzierende Struktur GalNAcβ-3-R trägt, um die Galβ-3GalNAcβ-3-R-Struktur zu bilden, wobei R ausgewählt ist aus der Gruppe, bestehend aus Galaktose, Galaktose-X, β-Galaktosiden wie z.B. Allyl-β-Galaktosid oder Propargyl-β-galaktosid, α-Galaktosiden, Globotriose, β-Globotriosid wie z.B. Allyl-β-Globotriosid oder Propargyl-β-Globotriosid, α-Globotriosid, wobei X als eine reaktive Gruppe definiert ist, die die kovalente Kopplung mit einem anderen Molekül ermöglicht, darin eingeschlossen Amino-, Azid- und Nitrophenylgruppen.

2. Verwendung nach Anspruch 1, um ein Oligosaccharid herzustellen, ausgewählt aus Galβ-3GalNAcβ-3Gal, Galβ-3GalNAcβ-3Galα-X, Galβ-3GalNAcβ-3Galβ-X, Galβ-3GalNAcβ-3Galβ-allyl, Galβ-3GalNAcβ-3Galβ-propargyl, Galβ-3GalNAcβ-3Galα-4Galβ-4Gal (Globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Galβ-4Galβ-allyl, und Galβ-3GalNAcβ-3Galα-4Galβ-4Galβ-propargyl.

3. Verwendung eines Mikroorganismus, umfassend ein heterologes *lgtD*-Gen von Haemophilus influenzae von SEQ ID Nr. 3 oder eine Sequenz mit mindestens 80 % Identität damit, um ein Oligosaccharid herzustellen, ausgewählt aus Galβ-3GalNAcβ-3Gal, Galβ-3GalNAcβ-3Galα-X, Galβ-3GalNAcβ-3Galβ-X, Galβ-3GalNAcβ-3Galβ-allyl, Galβ-3GalNAcβ-3Galβ-propargyl, Galβ-3GalNAcβ-3Galα-4Galβ-4Gal (Globopentaose), Galβ-3GalNAcβ-3Galα-4Galβ-4Gala-X, Galβ-3GalNAcβ-3Galα-4Galβ-4Galβ-X, Galβ-3GalNAcβ-3Galα-4Galβ-4Galβ-allyl, und Galβ-3GalNAcβ-3Galα-4Galβ-4Galβ-propargyl.

4. Verwendung nach Anspruch 3 eines Mikroorganismus, der *LacY*+, *LacZ-, melA*- ist und ein heterologes *lgtD-*Gen von Haemophilus influenzae von SEQ ID Nr. 3 oder eine Sequenz mit mindestens 80 % Identität damit umfasst und β-3 GalNAc-Transferase codiert und ein *wbpP,* das für UDP-GlcNAc-C4-Epimerase codiert, wie z.B. das *Pseudomonas aeruginos awbpP*-Gen oder ein *gne*-Gen, das für eine UDP-Glucose-4-Epimerase codiert, wie z.B. das *gne-*Gen von *C. jejuni* Stamm NCTC 11168 von SEQ ID Nr. 9 zur Herstellung von Galβ-3GalNAcβ-3Galα-4Galβ-4Gal (Globopentaose).

5. Verfahren zur Herstellung von Galβ-3GalNAcβ-3Galα-4Galβ-4Gal (Globopentaose), umfassend den Schritt des Kultivierens eines Mikroorganismus in einem Kulturmedium, umfassend Globotriose (Galα-4Galβ-4Glc), wobei der Mikroorganismus ein heterologes *wbpP* umfasst, das für UDP-GlcNAc-C4-Epimerase codiert, wie z.B. das *Pseudomonas aeruginos awbpP*-Gen oder ein *gne*-Gen, das für eine UDP-Glucose 4-Epimerase codiert, wie z.B. das *gne-*Gen von *C. jejuni* Stamm NCTC 11168 von SEQ ID Nr. 9 und ein heterologes *lgtD*-Gen von Haemophilus influenzae von SEQ ID Nr. 3 oder eine Sequenz mit mindestens 80 % Identität damit umfasst und β-3 GalNAc-Transferase codiert.

6. Verfahren zur Herstellung von Galβ-3GalNAcβ-3Galα-4Galβ-4Gal (Globopentaose), umfassend den Schritt des Kultivierens eines Mikroorganismus in einem Kulturmedium, umfassend Laktose, wobei der Mikroorganismus ein heterologes *lgtC*-Gen umfasst, das α-1,4-Gal-Transferase codiert, das einen Galaktoseanteil von UDP-Gal an die Laktose überträgt, um Globotriose (Galα-4Galβ-4Glc) zu bilden, ein heterologes *lgtD-*Gen von Haemophilus influenzae von SEQ ID Nr. 3 oder eine Sequenz mit mindestens 80 % Identität damit umfasst und β-3 GalNAc-Transferase codiert, und ein heterologes *wbpP,* das für UDP-GlcNAc-C4-Epimerase codiert, wie z.B. das *Pseudomonas aeruginos awbpP*-Gen oder ein *gne*-Gen, das für eine UDP-Glucose 4-Epimerase codiert, wie z.B. das gne-Gen von *C. jejuni* Stamm NCTC 11168 von SEQ ID Nr. 9.

7. Verfahren nach Anspruch 5 oder 6, wobei das *lgtD-*Gen einen GalNAc-Anteil von UDP-GalNAc an Globotriose überträgt, um Globotetraose (GalNAcβ-3-Galα-4Galβ-4Glc) zu bilden, ebenso wie einen Galaktoseanteil von UDP-Gal an Globotetraose, um Globopentaose (Galβ-3GalNAcβ-3Galα-4Galβ-4Gal) zu bilden.

8. Verfahren nach Anspruch 5, wobei Globotriose aus einem Verfahren erhalten wird, umfassend Kultivieren eines Mikroorganismus, der *LacY*+ (β-Galaktosidepermease), *LacZ*- (β Galaktosidase) und *MelA-*(α-Galaktosidase) ist, in einem Kulturmedium, umfassend Laktose, wobei der Mikroorganismus ein heterologes *lgtC*-Gen umfasst, das a-1,4-Gal-Transferase codiert, das einen Galaktoseanteil von UDP-Gal an die Laktose überträgt, um Globotriose (Galα-4Galβ-4Glc) zu bilden, und wobei Laktose überschüssig im Kulturmedium vorhanden ist.

9. Verfahren nach Anspruch 8, wobei die Kultur vor der Erschöpfung von Laktose beendet wird und Globotriose aus dem Kulturmedium extrahiert wird.

10. Verfahren nach Anspruch 8, wobei das *LgtC-*Gen von *Neisseria meningititis* ist.

11. Verfahren nach einem der Ansprüche 5 bis 8, wobei der Mikroorganismus LacY+ (β-Galaktosidepermease), *LacZ-* (β Galaktosidase) und *melA-* (α-Galaktosidase) ist.

12. Verfahren nach einem der Ansprüche 5 bis 8, wobei die Mikroorganismen rekombinante *E*. *coli*-Stämme sind.

13. Verfahren nach einem der Ansprüche 5 bis 8, wobei Glycerol in den Medien der Mikroorganismen als Kohlenstoff und Energiequelle verwendet wird.

14. Satz von zwei Mikroorganismen, umfassend den Mikroorganismus, wie in Anspruch 5 definiert, und einen Mikroorganismus, wie in Anspruch 8 definiert.

15. Zellkultur, umfassend Globotriose und einen Mikroorganismus von Anspruch 5.

16. Verwendung eines Mikroorganismus nach Anspruch 5 oder 6, um eine Globopentaose mit hoher Ausbeute in Zellkultur zu erzeugen.

17. Verwendung nach Anspruch 1 des *lgtD*-Gens von *Haemophilus influenzae* von SEQ ID Nr. 3 oder einer Sequenz, die mindestens 80 % Identität damit aufweist und β-3 GalNAc-Transferase codiert, um die Übertragung von einem Galaktoseanteil von UDP-Gal an Globotetraose zu katalysieren, um Globopentaose (β-3 Gal Transferaseaktivität) zu bilden.

18. Verfahren nach Anspruch 5 oder 6, wobei der Mikroorganismus weiter ein heterologes *futC*-Gen umfasst, das eine α-2-Fucosyltransferase codiert, um einen Fucoseanteil von GDP-Fuc an Globopentaose zu übertragen, um Globo-H-Hexasaccharid (Fucα-2Galβ-3GalNAcβ-3Galα-4Galβ-4Gal) zu bilden.

19. Mikroorganismus, wie in Anspruch 18 definiert, der *LacY+,* (optional *MelA-, manXXZ+*)*, manA⁻* ist, und der ein heterologes *lgtD*-Gen von *Haemophilus influenzae* von SEQ ID Nr. 3 oder eine Sequenz mit mindestens 80 % Identität damit (β-3 GalNAc-Transferase) umfasst, ein heterologes *wbpP,* das für UDP-GlcNAc-C4-Epimerase codiert, wie z.B. das *Pseudomonas aeruginosa wbpP*-Gen oder ein *gne-*Gen*,* das für eine UDP-Glucose-4-Epimerase codiert, wie z.B. das *gne-*Gen von C. *jejuni* Stamm NCTC 11168 von SEQ ID Nr. 9 und ein heterologes *futC*-Gen (α-2-Fucosyltransferase), wie z.B. das *Helicobacter pylori*-Gen *futC* von SEQ ID Nr. 5.

20. Satz von zwei Mikroorganismen, umfassend den Mikroorganismus, wie in Anspruch 19 definiert, und einen Mikroorganismus, umfassend ein heterologes *lgtC-*Gen, das α-1,4-GalTransferase codiert, und das LacY+ (β-Galaktosidepermease), *LacZ-* (β Galaktosidase) und *MelA-* (α-Galaktosidase) ist.

21. Verfahren nach Anspruch 5 oder 6, wobei der Mikroorganismus weiter ein Gen umfasst, das die CMP-NeuAc-Synthase codiert, wie z.B. ein Gen, das die CMP-NeuAc-Synthase von *N. meningitides* codiert, und ein heterologes Gen, das die α-3-Sialyltransferase codiert, wie z.B. das α-3-Sialyltransferase-Gen von *N. meningitidis* von SEQ ID Nr. 7, das die Übertragung eines Sialylanteils von einem aktivierten Sialsäuremolekül an Globopentaose katalysiert, um Sialosylgalaktosylglobosid (SGG) hexasaccharid (NeuAcα-3Galβ-3GalNAcβ-3Galα-4Galβ-4Gal) zu bilden.

22. Verfahren nach Anspruch 21, wobei NeuAc dem Medium nach der gesamten Umwandlung von Globotriose zu Globopentaose zugegeben wird.

23. Mikroorganismus, wie in Anspruch 21 definiert, der *LacY+*, *MelA-, nanT+, nanA⁻* ist, und der ein heterologes *lgtD*-Gen von *Haemophilus influenzae* von SEQ ID Nr. 3 oder eine Sequenz mit mindestens 80 % Identität damit (β-3 GalNAc-Transferase) umfasst, ein heterologes *wbpP,* das für UDP-GlcNAc-C4-Epimerase codiert, wie z.B. das *Pseudomonas aeruginosa wbpP*-Gen oder ein *gne*-Gen, das für eine UDP-Glucose 4-Epimerase codiert, wie z.B. das *gne-*Gen von *C. jejuni* Stamm NCTC 11168 von SEQ ID Nr. 9 und ein heterologes Gen, das die α-3 Sialyltransferase codiert, wie z.B. das α-3-Sialyltransferase-Gen von N. meningitidis von SEQ ID Nr. 7.

24. Zellkulturmedium, umfassend den Mikroorganismus, wie in Anspruch 23 definiert, und Sialylsäure.

25. Satz von zwei getrennten Mikroorganismen, umfassend den Mikroorganismus, wie in Anspruch 23 definiert, und einen Mikroorganismus, umfassend ein heterologes *lgtC-*Gen, das α-1,4-Gal-Transferase codiert, und das LacY+ (β-Galaktosidepermease), *LacZ-* (β Galaktosidase) und *MelA-* (α-Galaktosidase) ist.

26. Verfahren zur Herstellung eines Oligosaccharids, umfassend ein Galabiosemotiv (Gala-4Gal), bezeichnet als Globoside, ausgewählt aus der Gruppe, bestehend aus Globopentaose, und Galaktosyl-Globoside, umfassend Globo-H-hexasaccharid, Sialosylgalaktosylglobosid (SGG) hexasaccharid, umfassend den Schritt, bestehend aus dem Kultivieren eines Mikroorganismus, wie in Anspruch 5, 6, 19 oder 23 definiert.

27. Zellkultur, umfassend Globotriose in einer Konzentration von 1 bis 10 g.L ¹ und einen Mikroorganismus, wie in Anspruch 5, 19 oder 23 definiert.

28. Verwendung nach Anspruch 1 eines heterologen *lgtD-*Gens von *Haemophilus influenzae* von SEQ ID Nr. 3, oder einer Sequenz mit mindestens 80 % Identität damit, als einer Gal-Transferase in Anwesenheit von GalNAcβ-3Gal, um das SSEA-3-Antigen (Galβ-3GalNAcβ-3Gal) zu bilden.

29. Verfahren zur Herstellung eines Oligosaccharids, umfassend das Motiv GalNAcβ-3Gal, umfassend die Kultivierung eines Mikroorganismus, der *galP* (galaktose permease), *LacZ-* (β galaktosidase), *MelA-* (α-galaktosidase) und *wbpP* ist, der für UDP-GlcNAc-C4-Epimerase codiert, wie z.B. das *Pseudomonas aeruginosa* wbpP-Gen; oder ein gne-Gen, das für eine UDP-Glucose 4-Epimerase codiert, wie z.B. das gne-Gen von *C. jejuni* Stamm NCTC 11168 von SEQ ID Nr. 9. in einem Kulturmedium, umfassend Galaktose, wobei:
• der Mikroorganismus ein heterologes *lgtD*-Gen von *Haemophilus influenzae* von SEQ ID Nr. 3 oder eine Sequenz mit mindestens 80 % Identität damit umfasst und α-1,4-Gal GalNAc-Transferase codiert, die einen GalNAc-Rest an Galaktose überträgt, um ein Oligosaccharid zu bilden, das GalNAcβ-3Gal umfasst, und
• das *lgtD*-Genprodukt, das weiter einen Galaktoseanteil von UDP-Gal an GalNAcβ-3Gal übertragen kann, um das SSEA-3-Antigen (Galβ-3GalNAcβ-3Gal) zu bilden.

30. Verfahren nach Anspruch 29, das auf die Herstellung des terminalen Tetrasaccharidepitops des Globo-H-Antigens (Fuca-2Galβ-3GalNAcβ-3Gal) ausgedehnt ist, und wobei der Mikroorganismus weiter ein heterologes *futC-*Gen umfasst, das eine α-2-Fucosyltransferase codiert, um einen Fucoseanteil von GDP-Fuc an Galβ-3GalNAcβ-3Gal zu übertragen, um Fucα-2Galβ-3GalNAcβ-3Gal zu bilden.

31. Mikroorganismus, wie in Anspruch 29 definiert.

32. Mikroorganismus, wie in Anspruch 30 definiert.

33. Kulturmedium, umfassend Galaktose und einen Mikroorganismus nach Anspruch 31 oder 32.

## Revendications

1. Utilisation de la glycosyltransférase codée par le gène *lgtD* provenant d'*Haemophilus influenzae* de SEQ ID N° 3 ou d'une séquence ayant au moins 80 % d'identité avec celle-ci, comme β1,3-galactosyltransférase pour catalyser le transfert d'un résidu galactose à partir d'UDP-Gal vers un accepteur portant la structure terminale non réductrice GalNAcβ-3-R pour former la structure Galβ-3GalNAcβ-3-R, dans laquelle R est sélectionné dans le groupe consistant en le galactose, le galactose-X, les β-galactosides tels que l'allyl-β-galactoside ou le propargyl-β-galactoside, les α-galactosides, le globotriose, un β-globotrioside tel que l'allyl-β-globotrioside ou le propargyl-β-globotrioside, le α-globotrioside, X étant défini comme étant un groupe réactif permettant un couplage covalent avec une autre molécule, notamment les groupes amino, azide et nitrophényle.

2. Utilisation selon la revendication 1, pour produire un oligosaccharide sélectionné parmi Galβ-3GalNAcβ-3Gal, Galβ-3GalNAcβ-3Galα-X, Galβ-3GalNAcβ-3Galβ-X, Galβ-3GalNAcβ-3Galβ-allyle, Galβ-3GalNAcβ-3Galβ-propargyle, Galβ-3GalNAcβ-3Galα-4Galβ-4Gal (globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Galβ-4Galβ-allyle et Galβ-3GalNAcβ-3Galα-4Galβ-4Galβ-propargyle.

3. Utilisation d'un micro-organisme comprenant un gène *lgtD* hétérologue provenant d'*Haemophilus influenzae* de SEQ ID N° 3 ou une séquence ayant au moins 80 % d'identité avec celle-ci, pour produire un oligosaccharide sélectionné parmi Galβ-3GalNAcβ-3Gal, Galβ-3GalNAcβ-3Galα-X, Galβ-3GalNAcβ-3Galβ-X, Galβ-3GalNAcβ-3Galβ-allyle, Galβ-3GalNAcβ-3Galβ-propargyle, Galβ-3GalNAcβ-3Galα-4Galβ-4Gal (globopentaose), Galβ-3GalNAcβ-3Galα-4Galβ-4Galα-X, Galβ-3GalNAcβ-3Galα-4Galβ-4Galβ-X, Galβ-3GalNAcβ-3Galα-4Galβ-4Galβ-allyle et Galβ-3GalNAcβ-3Galα-4Galβ-4Galβ-propargyle.

4. Utilisation selon la revendication 3 d'un micro-organisme qui est *LacY*+, *LacZ-, melA-* et qui comprend un gène *lgtD* hétérologue provenant d'*Haemophilus influenzae* de SEQ ID N° 3 ou une séquence ayant au moins 80 % d'identité avec celle-ci et codant une β-3-GalNAc transférase, et un *wbpP* codant une UDP-GlcNAc-C4 épimérase, tel que le gène *wbpP* de *Pseudomonas aeruginosa,* ou un gène *gne* codant une UDP-glucose 4-épimérase, tel que le gène *gne* de la souche NCTC 11168 de *C. jejuni* de SEQ ID N° 9 pour produire Galβ-3GalNAcβ-3Galα-4Galβ-4Gal (globopentaose).

5. Méthode de production de Galβ-3GalNAcβ-3Galα-4Galβ-4Gal (globopentaose), comprenant l'étape de culture d'un micro-organisme dans un milieu de culture comprenant du globotriose (Galα-4Galβ-4Glc), dans laquelle ledit micro-organisme comprend un *wbpP* hétérologue codant une UDP-GlcNAc-C4 épimérase, tel que le gène *wbpP* de *Pseudomonas aeruginosa,* ou un gène *gne* codant une UDP-glucose 4-épimérase, tel que le gène *gne* de la souche NCTC 11168 de C. *jejuni* de SEQ ID N° 9 et un gène *lgtD* hétérologue provenant d'*Haemophilus influenzae* de SEQ ID N° 3 ou une séquence ayant au moins 80 % d'identité avec celle-ci et codant une β-3-GalNAc transférase.

6. Méthode de production de Galβ-3GalNAcβ-3Galα-4Galβ-4Gal (globopentaose), comprenant l'étape de culture d'un micro-organisme dans un milieu de culture comprenant du lactose, dans laquelle ledit micro-organisme comprend un gène *lgtC* hétérologue codant une α-1,4-Gal-transférase qui transfert un résidu galactose à partir d'UDP-Gal vers le lactose pour former le globotriose (Galα-4Galβ-4Glc), un gène *lgtD* hétérologue provenant d'*Haemophilus influenzae* de SEQ ID N° 3 ou une séquence ayant au moins 80 % d'identité avec celle-ci et codant une β-3-GalNAc transférase, et un *wbpP* hétérologue codant une UDP-GlcNAc-C4 épimérase, tel que le gène *wbpP* de *Pseudomonas aeruginosa,* ou un gène *gne* codant une UDP-glucose 4-épimérase, tel que le gène *gne* de la souche NCTC 11168 de *C. jejuni* de SEQ ID N° 9.

7. Méthode selon la revendication 5 ou 6, dans laquelle le gène *lgtD* transfert un résidu GalNAc à partir d'UDP-GalNAc vers le globotriose pour former le globotétraose (GalNAcβ-3-Galα-4Galβ-4Glc) ainsi qu'un résidu galactose à partir d'UDP-Gal vers le globotétraose pour former le globopentaose (Galβ-3GalNAcβ-3Galα-4Galβ-4Gal).

8. Méthode selon la revendication 5, dans laquelle le globotriose est obtenu à partir d'une méthode comprenant la culture d'un micro-organisme qui est *LacY*+ (β-galactoside perméase), *LacZ-* (β-galactosidase), et *MelA-* (α-galactosidase) dans un milieu de culture comprenant du lactose, dans laquelle ledit micro-organisme comprend un gène *lgtC* hétérologue codant une α-1,4-Gal-transférase qui transfert un résidu galactose à partir d'UDP-Gal vers le lactose pour former le globotriose (Galα-4Galβ-4Glc) et dans laquelle le lactose est en excès dans le milieu de culture.

9. Méthode selon la revendication 8, dans laquelle la culture est terminée avant l'épuisement du lactose et le globotriose est extrait du milieu de culture.

10. Méthode selon la revendication 8, dans laquelle le gène *LgtC* provient de *Neisseria meningititis*.

11. Méthode selon l'une quelconque des revendications 5 à 8, dans laquelle ledit micro-organisme est *LacY*+ (β-galactoside perméase), *LacZ*- (5-galactosidase), *melA-* (α-galactosidase).

12. Méthode selon l'une quelconque des revendications 5 à 8, dans laquelle les micro-organismes sont des souches recombinantes d'*E*. *coli.*

13. Méthode selon l'une quelconque des revendications 5 à 8, dans laquelle du glycérol est utilisé dans les milieux desdits micro-organismes comme source de carbone et d'énergie.

14. Ensemble de deux micro-organismes séparés, comprenant ledit micro-organisme tel que défini dans la revendication 5 et un micro-organisme tel que défini dans la revendication 8.

15. Culture cellulaire comprenant du globotriose et un micro-organisme selon la revendication 5.

16. Utilisation d'un micro-organisme selon la revendication 5 ou 6 pour produire du globopentaose à un rendement élevé dans une culture cellulaire.

17. Utilisation selon la revendication 1 du gène *lgtD* provenant d'*Haemophilus influenzae* de SEQ ID N° 3 ou d'une séquence ayant au moins 80 % d'identité avec celle-ci et codant une β-3-GalNAc transférase, pour catalyser le transfert d'un résidu galactose à partir d'UDP-Gal vers le globotétraose pour former du globopentaose (activité β-3-Gal transférase).

18. Méthode selon la revendication 5 ou 6, dans laquelle ledit micro-organisme comprend en outre un gène *futC* hétérologue codant une α-2-fucosyltransférase pour transférer un résidu fucose à partir du GDP-Fuc vers le globopentaose pour former un globo-H hexasaccharide (Fucα-2Galβ-3GalNAcβ-3Galα-4Galβ-4Gal).

19. Micro-organisme tel que défini dans la revendication 18, qui est *LacY*+, (facultativement *MelA-, manXXZ+), manA*- et qui comprend un gène *lgtD* hétérologue provenant d'*Haemophilus influenzae* de SEQ ID N° 3 ou une séquence ayant au moins 80 % d'identité avec celle-ci (β-3-GalNAc transférase), un *wbpP* hétérologue codant une UDP-GlcNAc-C4 épimérase, tel que le gène *wbpP* de *Pseudomonas aeruginosa,* ou un gène *gne* codant une UDP-glucose 4-épimérase, tel que le gène *gne* de la souche NCTC 11168 de C. *jejuni* de SEQ ID N° 9 et un gène *futC* hétérologue (α-2-fucosyltransférase), tel que le gène *futC* d'*Helicobacter pylori* de SEQ ID N° 5.

20. Ensemble de deux micro-organismes séparés, comprenant ledit micro-organisme tel que défini dans la revendication 19 et un micro-organisme comprenant un gène *lgtC* hétérologue codant une α-1,4-Gal transférase et qui est *LacY*+ (β-galactoside perméase), *LacZ-*(β-galactosidase) et *MelA-* (α-galactosidase).

21. Méthode selon la revendication 5 ou 6, dans laquelle ledit micro-organisme comprend en outre un gène codant la CMP-NeuAc synthase, tel qu'un gène codant la CMP-NeuAc synthase provenant de *N. meningitidis,* et un gène hétérologue codant une α-3-sialyltransférase, tel que le gène de la α-3-sialyl-transférase provenant de *N. meningitidis* de SEQ ID N° 7, qui catalyse le transfert d'un résidu sialyle à partir d'une molécule d'acide sialique activée vers le globopentaose pour former un sialosyl galactosyl globoside (SGG) hexasaccharide (NeuAcα-3Galβ-3GalNAcβ-3Galα-4Galβ-4Gal).

22. Méthode selon la revendication 21, dans laquelle NeuAc est ajouté au milieu après la conversion totale du globotriose en globopentaose.

23. Micro-organisme tel que défini dans la revendication 21, qui est *LacY*+, *MelA-, nanT*+, *nanA*- et qui comprend un gène *lgtD* hétérologue provenant d'*Haemophilus influenzae* de SEQ ID N° 3 ou une séquence ayant au moins 80 % d'identité avec celle-ci (β-3-GalNAc transférase), un *wbpP* hétérologue codant une UDP-GlcNAc-C4 épimérase, tel que le gène *wbpP* de *Pseudomonas aeruginosa,* ou un gène *gne* codant une UDP-glucose 4-épimérase, tel que le gène *gne* de la souche NCTC 11168 de *C. jejuni* de SEQ ID N° 9 et un gène hétérologue pour une α-3-sialyltransférase, tel que le gène provenant de *N. meningitidis* de SEQ ID N° 7.

24. Milieu de culture cellulaire, comprenant le micro-organisme tel que défini dans la revendication 23 et de l'acide sialique.

25. Ensemble de deux micro-organismes séparés, comprenant ledit micro-organisme tel que défini dans la revendication 23 et un micro-organisme comprenant un gène *lgtC* hétérologue codant une α-1,4-Gal transférase et qui est *LacY*+ (β-galactoside perméase), *LacZ-*(β-galactosidase) et *MelA-* (α-galactosidase).

26. Méthode de production d'un oligosaccharide comprenant le motif galabiose (Gala-4Gal), dénommé globosides, sélectionné dans le groupe consistant en le globopentaose et les galactosyl-globosides incluant un globo-H hexasaccharide, un sialosyl galactosyl globoside (SGG) hexasaccharide, comprenant l'étape consistant à cultiver un micro-organisme tel que défini dans la revendication 5, 6, 19 ou 23.

27. Culture cellulaire comprenant le globotriose à une concentration de 1 à 10 g.l⁻¹ et un micro-organisme tel que défini dans la revendication 5, 19 ou 23.

28. Utilisation selon la revendication 1 d'un gène *lgtD* hétérologue provenant d'*Haemophilus influenzae* de SEQ ID N° 3 ou d'une séquence ayant au moins 80 % d'identité avec celle-ci, comme une Gal transférase en présence de GalNAcβ-3Gal pour former l'antigène SSEA-3 (Galβ-3GalNAcβ-3Gal).

29. Méthode de production d'un oligosaccharide comprenant le motif GalNAcβ-3Gal, comprenant la culture d'un micro-organisme qui est *galP* (galactose perméase), *LacZ-* (β-galactosidase), *MelA-* (α-galactosidase) et *wbpP* codant une UDP-GlcNAc-C4 épimérase, tel que le gène wbpP de *Pseudomonas aeruginosa ;* ou un gène *gne* codant une UDP-glucose 4-épimerase, tel que le gène *gne* de la souche NCTC 11168 de *C. jejuni* de SEQ ID N° 9, dans un milieu de culture comprenant du galactose, dans laquelle :
• ledit micro-organisme comprend un gène *lgtD* hétérologue provenant d'*Haemophilus influenzae* de SEQ ID N° 3 ou une séquence ayant au moins 80 % d'identité avec celle-ci et codant une α-1,4-Gal transférase qui transfert un résidu GalNAc vers le galactose pour former un oligosaccharide comprenant GalNAcβ-3Gal, et
• le produit du gène *lgtD* a permis de transférer en outre un résidu galactose à partir d'UDP-Gal vers GalNAcβ-3Gal pour former l'antigène SSEA-3 (Galβ-3GalNAcβ-3Gal).

30. Méthode selon la revendication 29, qui est étendue à la production de l'épitope tétra-saccharidique terminal de l'antigène globo-H (Fucα-2Galβ-3GalNAcβ-3Gal) et dans laquelle le micro-organisme comprend en outre un gène *futC* hétérologue codant une α-2-fucosyltransférase pour transférer un résidu fucose à partir du GDP-Fuc vers Galβ-3GalNAcβ-3Gal pour former Fucα-2Galβ-3GalNAcβ-3Gal.

31. Micro-organisme tel que défini dans la revendication 29.

32. Micro-organisme tel que défini dans la revendication 30.

33. Milieu de culture comprenant du galactose et un micro-organisme selon la revendication 31 ou 32.
